# EUROPEAN PATENT APPLICATION

(11) **EP 3 266 784 A1**
(43) Date of publication of application: **10.01.2018**
(21) Application number: 16305672.4
(22) Date of filing: 08.06.2016
(51) Int. Cl.: C07D 473/34, A61K 31/52, A61P 35/00, A61P 9/00, A61P 11/00

(54) **PROTEIN ARGININE N-METHYLTRANSFERASES INHIBITORS AND USES THEREOF**

(71) Applicant: PIERRE FABRE MEDICAMENT, 92100 Boulogne-Billancourt (FR); CENTRE NATIONAL DE LA RECHERCHE SCIENTIFIQUE (CNRS), 75016 Paris (FR); UNIVERSITE DE STRASBOURG, 67000 Strasbourg (FR); INSTITUT NATIONAL DE LA SANTE ET DE LA RECHERCHE MEDICALE (INSERM), 75013 Paris (FR)
(72) Inventor: CAVARELLI, Jean, 67200 Strasbourg (FR); HALBY, Ludovic, 31400 Toulouse (FR); ARIMONDO, Paola Barbara, 31000 Toulouse (FR)
(74) Representative: Regimbeau

(57) **Abstract**

The present invention relates to a compound of following formula (I): 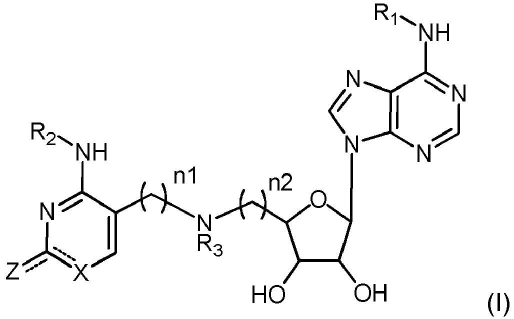 or a pharmaceutically acceptable salt and/or solvate thereof,
notably for use thereof as a drug, notably in the treatment of cancer, or as a PRMT inhibitor.

The present invention relates also to a pharmaceutical composition containing such a compound and to a method for the preparation of such a compound.

## Description

### Field of the Invention

The present invention relates to compounds useful as protein arginine *N-*methyltransferase (PRMT) inhibitors, notably in the treatment of cancer.

### Background of the Invention

Histone methyltransferases (HMT) are histone-modifying enzymes (e.g., histone-lysine *N*-methyltransferases and histone-arginine *N*-methyltransferases) that catalyze the transfer of one, two, or three methyl groups to lysine and arginine residues of histone proteins. The attachment of methyl groups occurs predominantly at specific lysine or arginine residues on histones H3 and H4. Two major types of histone methyltransferases exist, lysine-specific and arginine-specific. In both types of histone methyltransferases, cofactor *S-*adenosyl-L-methionine (SAM) serves as a cofactor and methyl donor group. In eukaryotic cells, the genome is tightly condensed into chromatin (composed of DNA and histone proteins) and its accessibility is regulated by epigenetic enzymes, such as histone methyltransferases. Histone methyltransferase does so by modifying histones at certain sites through methylation. Methylation of histones, in particular histone arginine methylation, is important biologically because epigenetic modification of chromatin determines gene expression, genomic stability, stem cell maturation, cell lineage development, genetic imprinting, DNA methylation, and cell mitosis.

Abnormal expression or activity of methylation-regulating enzymes has been noted in some types of human cancers, suggesting associations between histone methylation and malignant transformation of cells or formation of tumors. In recent years, epigenetic modification of the histone proteins, especially the methylation of the histone H3, has been an area of emerging research in cancer development. It is now accepted that, in addition to genetic aberrations, cancer can be initiated and maintained by epigenetic changes, in which gene expression is altered without genomic abnormalities. These epigenetic changes include loss or gain of methylations in both DNA and histone proteins.

Arginine methylation is an abundant cellular posttranslational modification occurring ubiquitously in eukaryotic organisms, in particular mammalian cells, which is catalyzed by a family of enzymes called the protein arginine methyltransferases (PRMTs) and is implicated in a number of cellular processes including transcription, DNA repair, RNA metabolism, signal transduction, protein-protein interactions and subcellular localization. Deregulation and aberrant expression of PRMTs are associated with various disease states, notably cancer. PRMTs have recently become the subject of investigation as therapeutic targets in drug discovery.

There are two different types of protein arginine methyltransferases (PRMTs) and both catalyze the addition of one or two methyl groups to the guanidino nitrogen atoms of arginine, resulting in either ω-NG-monomethylarginine (MMA), ω-NG, NG-asymmetric (ADMA) or ω-NG, N'G-symmetric dimethylarginine (SDMA) (Zakrzewicz et al., 2012, Int. J. Mol. Sci., 13, 12383-12400*).* In humans, PRMTs have been classified into two types, depending on their specific catalytic activities. Both types of enzymes first catalyze the formation of MMA as an intermediate. In a second step, type I enzymes (PRMT1, PRMT3, CARM1/PRMT4, PRMT6 and PRMT8) lead to the formation of ADMA, whereas the type II enzymes (PRMT5 and PRMT7) produce SDMA.

A number of recent studies have pointed to multifaceted roles of PRMT deregulation in disease development. PRMT-related pathologies include virus-related diseases, inflammatory response, cardiovascular disease, renal disease and diabetes, pulmonary disorders, and the most actively studied area, carcinogenesis *(*Hu et al., 2016, Expert Opinion on Investigational Drugs, 25(3), 335-358).

A number of increasing evidence of the specific involvement of PRMT1, CARM1, PRMT6 and PRMT7, which appear to promote tumorigenesis in breast cancer cells, has led to an increased interest into these four PRMTs as novel therapeutic targets in the treatment of breast cancer (Morettin et al., 2015, Mutagenesis, 30, 177-189). Further, elevated PRMT1 and PRMT6 expression has also been found in colon and bladder cancers, but also in various types of lung cancer including Small-cell carcinoma (SCLC) and Non-small-cell lung carcinoma (NSCLC) *(*Yang et al., 2013, Nature Reviews, 13, 37-50*; Zakrzewicz et al., 2012, supra).*

Further, PRMTs also have a broad spectrum of non-histone substrates, which are involved in crucial cellular regulations, such as transcription, RNA splicing, signal transduction, nuclear/cytoplasmic shuttling, and DNA repair and are also considered as potential targets useful in the treatment of endothelial dysfunction-related cardiovascular and pulmonary diseases, including coronary heart disease, pulmonary hypertension, and virus-related diseases *(*Wei et al., 2014, Cell Cycle 13:1, 32-41).
Inhibitors of histone arginine methyltransferases such as protein arginine methyltransferase 1 (PRMT1) and PRMT4 have also been identified *(*Sack et al., 2011, Biochem. J. 436, 331-339*;* Dowden et al., 2010, Bioorg. Med. Chem. Lett. 20, 2103-2105*;* Cheng et al., 2004, J. Biol. Chem. 279, 23892-23899), supporting the possibility to achieve some PRMT inhibition through the use of small molecules.

There is thus a need for the development of novel PRMT inhibitors.

### Summary of the Invention

The inventors of the present invention have discovered new compounds that exhibit inhibitory activities against PRMT, in particular CARM1, PRMT6 and 7 and therefore which can be useful as PRMT inhibitors.

The present invention concerns thus a compound of the following formula (I): 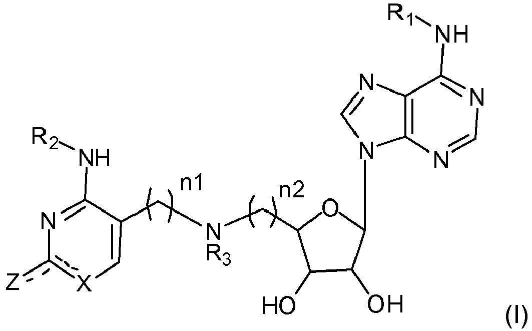 or a pharmaceutically acceptable salt and/or solvate thereof, wherein:
- -̅-̅-̅-̅-̅ represents a single bond or a double bond provided that the two adjacent -̅-̅-̅-̅-̅ bonds do not represent simultaneously a double bond,
- n1 and n2 represents, independently of each other, an integer comprised between 0 and 4, notably between 1 and 4, in particular being 1 or 2,
- X represents N when -̅-̅-̅-̅-̅X represents a double bond =X or NR₄ when -̅-̅-̅-̅-̅X represents a single bond -X,
- Z represents O or S when -̅-̅-̅-̅-̅Z represents a double bond =Z or H or NR₅R₆ when -̅-̅-̅-̅-̅Z represents a single bond -Z,
- R₁, R₂, R₅ and R₆ represent, independently of one another, H, (C₁-C₆)alkyl, alkylaryl or arylalkyl,
- R₃ represents H, or a (C₁-C₆)alkyl optionally substituted, and
- R₄ represents H, or a (C₁-C₆ alkyl) optionally substituted with (C₁-C₆)alkoxy, (C₁-C₆)haloalkoxy, aryloxy or haloaryloxy.

### Detailed description of the Invention

The compound according to the present invention can be in the form of a stereoisomer or a mixture of stereoisomers, such as a mixture of enantiomers, notably a racemic mixture.

The term "stereoisomers" used in this invention refers to configurational stereoisomers and more particularly to optical isomers.

The optical isomers result from the different position in space of substituents or lone pair of electrons on an atom (such as a carbon or sulphur atom) comprising four different substituents (including potentially a lone pair of electron). This atom thus represents a chiral or asymmetric center. Optical isomers that are not mirror images of one another are thus designated as "diastereoisomers," and optical isomers, which are non-superimposable mirror images are designated as "enantiomers".

An equimolar mixture of two enantiomers of a chiral compound is designated as a racemic mixture or racemate.

For the purpose of the invention, the term "pharmaceutically acceptable" is intended to mean what is useful to the preparation of a pharmaceutical composition, and what is generally safe and non-toxic, for a pharmaceutical use.

The term "pharmaceutically acceptable salt and/or solvate" is intended to mean, in the framework of the present invention, a salt or solvate of a compound which is pharmaceutically acceptable, as defined above, and which possesses the pharmacological activity of the corresponding compound.

The pharmaceutically acceptable salts comprise:
(1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L25 tartaric, tartaric, p-toluenesulfonic, trimethylacetic, and trifluoroacetic acid and the like, and
(2) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

Acceptable solvates for the therapeutic use of the compounds of the present invention include conventional solvates such as those formed during the last step of the preparation of the compounds of the invention due to the presence of solvents. As an example, mention may be made of solvates due to the presence of water (these solvates are also called hydrates) or ethanol.

The term "halogen", as used in the present invention, refers to a fluorine, bromine, chlorine or iodine atom.

The term "(C₁-C₆)alkyl", as used in the present invention, refers to a straight or branched monovalent saturated hydrocarbon chain containing from 1 to 6 carbon atoms including, but not limited to, methyl, ethyl, n-propyl, isopropyl, n-butyl, iso-butyl, sec-butyl, *t*-butyl, n-pentyl, n-hexyl, and the like.

The term "(C₂-C₆)alkenyl", as used in the present invention, refers to a straight or branched monovalent unsaturated hydrocarbon chain containing from 2 to 6 carbon atoms and comprising at least one double bond including, but not limited to, ethenyl, propenyl, butenyl, pentenyl, hexenyl and the like.

The term "(C₁-C₆)alkoxy", as used in the present invention, refers to a (C₁-C₆)alkyl chain as defined above linked to the rest of the molecule via an oxygen atom including, but not limited to, methoxy, ethoxy, n-propoxy, iso-propoxy, n-butoxy, iso-butoxy, sec-butoxy, *t*-butoxy, n-pentoxy, n-hexoxy, and the like. It can be a methoxy.

The term "(C₁-C₆)haloalkoxy" refers to a (C₁-C₆)alkoxy chain as defined above wherein one or more hydrogen atoms are replaced by a halogen atom selected from fluorine, chlorine, bromine or iodine, in particular bromine.

The term "(C₁-C₆)alkanediyl" as used in the present invention refers to a straight or branched divalent saturated hydrocarbon chain containing from 1 to 6 carbon atoms including, but not limited to, methanediyl (also called methylene), ethanediyl, propanediyl, isopropylidene, butanediyl, pentanediyl, hexanediyl, and the like. It can be in particular an isopropylidene.

The term "trialkylsilyl group", as used in the present invention, refers to a group -SiAlk₁Alk₂Alk₃ in which Alk₁, Alk₂ and Alk₃, identical or different, represent a (C₁-C₆)-alkyl group as defined above. For example, it can be a trimethylsilyl or triethylsilyl group.

The term "aryl" refers to an aromatic hydrocarbon group preferably comprising from 6 to 12 carbon atoms and comprising one or more fused rings, such as, for example, a phenyl or naphthyl group. Advantageously, it is a phenyl group.

The term "aryl-(C₁-C₆)alkyl" or "arylalkyl", as used in the present invention, refers to an aryl group as defined above bound to the molecule via a (C₁-C₆)alkyl group as defined above. In particular, it is a benzyl group.

The term "(C₁-C₆)alkyl-aryl" or "alkylaryl", as used in the present invention, refers to a (C₁-C₆)alkyl group as defined above bound to the molecule via an aryl group as defined above. In particular, it can be a tolyl group (CH₃Ph).

The term "aryloxy", as used in the present invention, refers to an aryl as defined above linked to the rest of the molecule via an oxygen atom including, but not limited to, phenoxy and naptoxy. Preferably it is a phenoxy.

The term "haloaryloxy" refers to an aryloxy as defined above wherein one or more hydrogen atoms are replaced by a halogen atom selected from fluorine, chlorine, bromine or iodine, in particular bromine. It can be a bromophenoxy, such as a para-bromophenoxy.

In the context of the present invention, "optionally substituted" means that the group in question is optionally substituted with one or more substituents which may be selected in particular from halogen, OR₇, NR₈R₉, COR₁₀, CO₂R₁₁ and CONR₁₂R₁₃ wherein R₇ to R₁₃ are, independently of one another, H or (C₁-C₆)alkyl.

The efficacy of a compound of the invention as a PRMT inhibitor can be tested in standard assays, such as notably described in the examples.

The efficacy of treatment of a cancer according to the invention can be measured for example, by monitoring the number or size of tumors (e.g. detected by imaging, and by the serial measurement of appropriate tumor-specific markers). Effective treatment is indicated by reduction in tumor number or size, and diminishing levels or maintenance of basal levels of at least one tumor specific marker. Successful outcome results in an increase of progression free survival time, and/or a decreased risk of relapse post-resection.

According to a particular embodiment, one of the -̅-̅-̅-̅-̅ bonds is a single bond and the other -̅-̅-̅-̅-̅ bond is a double bond. Thus, the compound according to the invention can be a compound of following formula (Ia) or formula (Ib), preferably a compound of formula (Ia): 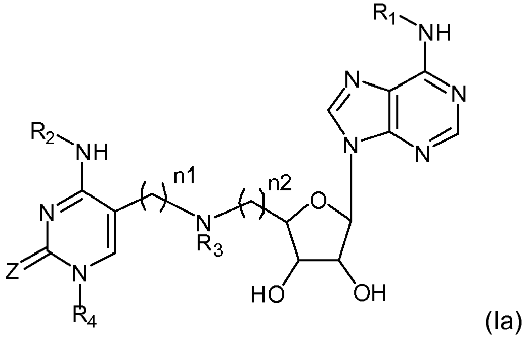 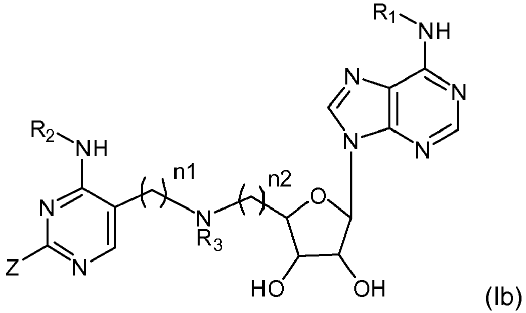 or a pharmaceutically acceptable salt and/or solvate thereof.

According to another particular embodiment, Z represents O and the compound according to the invention can be a compound of following formula (Ic): 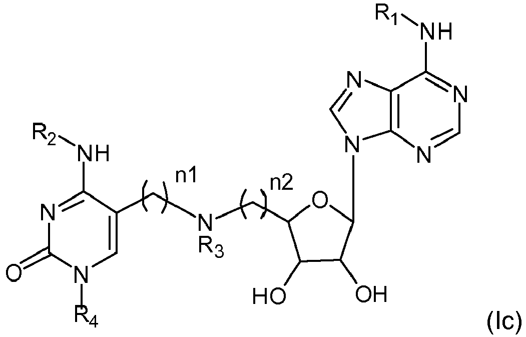 or a pharmaceutically acceptable salt and/or solvate thereof.

In formulas (I), (Ia), (Ib) and (Ic), the cycle 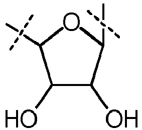 has advantageously the following stereochemistry:

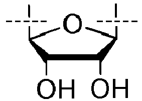

In a particular embodiment, n1 can represent 1, 2, 3 or 4, notably 1, 2, 3, notably 1 or 2, notably 1.

In another particular embodiment, n2 can represent 1, 2, 3 or 4, notably 1 or 2, notably 1.

In another particular embodiment, R₁, R₂, R₅ and R₆ advantageously represent, independently of one another, H or (C₁-C₆)alkyl, preferably H.

In another particular embodiment, Z represents advantageously O.

In another particular embodiment, R₃ represents notably H or a (C₁-C₆)alkyl (e.g. ethyl) optionally substituted with one or more substituents selected from halogen, OR₇, NR₈R₉, COR₁₀, CO₂R₁₁ and CONR₁₂R₁₃ wherein R₇ to R₁₃ are as defined previously.

R₃ represents in particular H or a (C₁-C₆)alkyl optionally substituted with one or more substituents selected from halogen, OR₇, NR₈R₉, COR₁₀, CO₂R₁₁ and CONR₁₂R₁₃.

R₃ represents advantageously H or a (C₁-C₆)alkyl optionally substituted with one or more substituents selected from OR₇, NR₈R₉, COR₁₀, CO₂R₁₁ and CONR₁₂R₁₃.

R₃ represents particularly H or a (C₁-C₆)alkyl (e.g. ethyl) optionally substituted with one or more substituents selected from NR₈R₉ and CO₂R₁₁.

In another particular embodiment, R₃ can represent H or CH₂CH₂CH(COO⁻) (NH₃⁺).

In another particular embodiment, R₄ represents notably H or a (C₁-C₆ alkyl) optionally substituted with a (C₁-C₆)alkoxy, (C₁-C₆)haloalkoxy, aryloxy or haloaryloxy; such as a (C₁-C₆ alkyl) (e.g. methyl or ethyl) optionally substituted with a (C₁-C₆)alkoxy (e.g. methyloxy), (C₁-C₆)haloalkoxy, aryloxy (e.g. phenyloxy) or haloaryloxy (e.g. bromo-phenyloxy).

In a further particular embodiment, R₄ represents in particular H or a (C₁-C₆ alkyl) optionally substituted with a (C₁-C₆)alkoxy, aryloxy or haloaryloxy; such as a (C₁-C₆ alkyl) optionally substituted with a (C₁-C₆)alkoxy, aryloxy or haloaryloxy.

In another further particular embodiment, R₄ represents advantageously H or a (C₁-C₆ alkyl) optionally substituted with a (C₁-C₆)alkoxy, aryloxy or bromoaryloxy; such as a (C₁-C₆ alkyl) optionally substituted with a (C₁-C₆)alkoxy, aryloxy or bromoaryloxy.

In another further particular embodiment, R₄ can be CH₂OCH₃, CH₂CH₂OPh or CH₂CH₂O(pBrPh).

According to a particular embodiment, the compound according to the present invention is a compound of formula (Ia), notably of formula (Ib), and preferably of formula (Ic), or a pharmaceutically acceptable salt and/or solvate thereof wherein:
- n1 and n2 represents, independently of each other, 1, 2, 3 or 4, preferably 1 or 2,
- R₁ and R₂ represent, independently of each other, H or (C₁-C₆)alkyl, preferably H,
- R₃ represents H or a (C₁-C₆)alkyl optionally substituted with one or more substituents selected from halogen, OR₇, NR₈R₉, COR₁₀, CO₂R₁₁ and CONR₁₂R₁₃; notably selected from OR₇, NR₈R₉, COR₁₀, CO₂R₁₁ and CONR₁₂R₁₃; in particular selected from NR₈R₉ and CO₂R₁₁, and
- R₄ represents H or a (C₁-C₆ alkyl) optionally substituted with a (C₁-C₆)alkoxy, (C₁-C₆)haloalkoxy, aryloxy or haloaryloxy; notably H or a (C₁-C₆ alkyl) optionally substituted with a (C₁-C₆)alkoxy, aryloxy or haloaryloxy.

According to another particular embodiment, the compound according to the present invention is a compound of formula (Ib), preferably of formula (Ic), or a pharmaceutically acceptable salt and/or solvate thereof wherein:
- n1 and n2 represents, independently of each other, 1 or 2,
- R₁ and R₂ represent H,
- R₃ represents H or a (C₁-C₆)alkyl optionally substituted with one or more substituents selected from OR₇, NR₈R₉, COR₁₀, CO₂R₁₁ and CONR₁₂R₁₃; in particular selected from NR₈R₉ and CO₂R₁₁, and
- R₄ represents H or a (C₁-C₆ alkyl) optionally substituted with a (C₁-C₆)alkoxy, (C₁-C₆)haloalkoxy, aryloxy or haloaryloxy; notably H or a (C₁-C₆ alkyl) optionally substituted with a (C₁-C₆)alkoxy, aryloxy or haloaryloxy.

In these two particular embodiments, the cycle 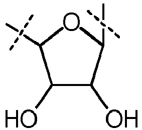 has advantageously the following stereochemistry:

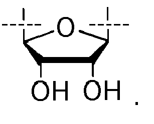

The compounds of the present invention may be selected from compounds A to F described in the experimental part below and the pharmaceutically acceptable salts and/or solvates thereof.

The present invention relates also to a compound of formula (I) or a pharmaceutically acceptable salt and/or solvate thereof as defined above, for use as a drug, notably intended for the prevention and/or treatment of a PRMT-related disorder, such as a cancer or an endothelial dysfunction-related cardiovascular or pulmonary disease.

The present invention also relates to the use of a compound of formula (I) or a pharmaceutically acceptable salt and/or solvate thereof as defined above, for the manufacture of a drug, notably intended for the prevention and/or treatment of a PRMT-related disorder, such as a cancer or an endothelial dysfunction-related cardiovascular or pulmonary disease.

The present invention also relates to a method for the prevention and/or treatment of a PRMT-related disorder, such as a cancer or an endothelial dysfunction-related cardiovascular or pulmonary disease comprising the administration to a person in need thereof of an effective dose of a compound of formula (I) or a pharmaceutically acceptable salt and/or solvate thereof as defined above.

According to the invention, the expression "PRMT-related disorders" includes any disorders related to the upregulation, high levels or deregulation of at least one PRMT and the treatment or prevention of which would benefit from the use of a PRMT inhibitor. For example, PRMT-related disorders includes cancers, cardiovascular and pulmonary diseases, in particular endothelial dysfunction-related cardiovascular or pulmonary diseases, such as coronary heart disease and pulmonary hypertension, and virus-related diseases. It can be in particular a cancer.

According to a particular embodiment, cancer may be selected from colon cancer, breast cancer, kidney cancer, liver cancer, pancreatic cancer, prostate cancer, bladder cancer, glioblastoma, lung cancer (e.g. non-small cell lung cancer), neuroblastoma, inflammatory myofibroblastic tumor, leukemia (e.g. acute myeloid leukemia, myelodysplastic syndrome or chronic myelomonocytic leukemia), melanoma, and lymphoma (e.g. diffuse B-cell lymphoma or anaplastic large-cell lymphoma).

According to a further particular embodiment cancer is breast cancer.

The present invention relates also to a compound of formula (I) or a pharmaceutically acceptable salt and/or solvate thereof as defined above, for use as a PRMT inhibitor, in particular as a PRMT1, PRMT4/CARM1, PRMT6 and/or PRMT7 inhibitor.

According to the invention, the expression "PRMT inhibitor" refers to molecules that are able to reduce or inhibit arginine methylation of histone and of non-histone proteins. Preferentially, the use of a PRMT inhibitor according to the invention makes it possible to suppress the activity of said PRMT. According to a particular aspect, a PRMT inhibitor of the present invention is able to inhibit at least one PRMT selected among PRMT1, PRMT4/CARM1, PRMT6 and PRMT7.

The present invention also relates to a pharmaceutical composition comprising at least one compound of formula (I) or a pharmaceutically acceptable salt and/or solvate thereof as defined above, and at least one pharmaceutically acceptable excipient.

The active ingredient may be administered in unit dosage forms of administration, in mixture with standard pharmaceutical carriers, to animals, in particular mammals, including humans. The pharmaceutical compositions according to the invention may be formulated notably for oral administration or parenteral administration (e.g. intravenous injection).

For oral administration, the pharmaceutical composition can be in a solid or liquid (solution or suspension) form.
A solid composition can be in the form of tablets, gelatin capsules, powders, granules and the like. In tablets, the active ingredient can be mixed with pharmaceutical vehicle(s) such as gelatin, starch, lactose, magnesium stearate, talc, gum arabic and the like before being compressed. The tablets may be coated, notably with sucrose or with other suitable materials, or they may be treated in such a way that they have a prolonged or delayed activity. In powders or granules, the active ingredient can be mixed or granulated with dispersing agents, wetting agents or suspending agents and with flavour correctors or sweeteners. In gelatine capsules, the active ingredient can be introduced into soft or hard gelatine capsules for example in the form of a powder or granules, such as mentioned previously.
A liquid composition can contain the active ingredient together with a sweetener, a taste enhancer or a suitable colouring agent in a solvent such as water. The liquid composition can also be obtained by suspending or dissolving a powder or granules, as mentioned above, in a liquid such as water, juice, milk, etc.. It can be for example a syrup or an elixir.

For parenteral administration, the composition can be in the form of an aqueous suspension or solution, which may contain dispersing agents and/or wetting agents. The composition is advantageously sterile. It can be in the form of an isotonic solution (in particular in comparison to blood).

The compounds of the invention as active ingredients may be used in doses ranging between 0.01 mg and 1000 mg per day, given in a single dose once per day or administered in several doses throughout the day, for example twice a day in equal doses. The dose administered per day advantageously is between 5 mg and 500 mg, even more advantageously between 10 mg and 200 mg. It may be necessary to use doses outside these ranges as determined by the person skilled in the art.

The pharmaceutical compositions according to the invention may further comprise at least one other active ingredient, such as an anticancer agent.

The present invention relates also to a pharmaceutical composition comprising:
(i) at least one compound of formula (I) or a pharmaceutically acceptable salt and/or solvate thereof as defined above, and
(ii) at least one other active ingredient, such as an anticancer agent,
as a combination product for simultaneous, separate or sequential use.

The present invention also relates to a pharmaceutical composition as defined above for use as a drug, notably intended for the prevention and/or treatment of a PRMT-related disorder, in particular cancer.

The present invention also relates to the use of a pharmaceutical composition as defined above, for the manufacture of a drug, notably intended for the prevention and/or treatment of a PRMT-related disorder, in particular cancer.

The present invention also relates to a method for the prevention and/or treatment of a PRMT-related disorder, in particular cancer, comprising the administration to a person in need thereof of an effective dose of a pharmaceutical composition as defined above.

The cancer may be more particularly in this case colon cancer, breast cancer, kidney cancer, liver cancer, pancreatic cancer, prostate cancer, glioblastoma, lung cancer (e.g. non-small cell lung cancer), neuroblastoma, inflammatory myofibroblastic tumor, leukemia (e.g. acute myeloid leukemia, myelodysplastic syndrome or chronic myelomonocytic leukemia), melanoma, or lymphoma (e.g. diffuse B-cell lymphoma or anaplastic large-cell lymphoma).

According to a particular aspect, compounds of the invention and pharmaceutical formulations thereof can be administered alone or in combination with a co-agent useful for treating, and/or stabilizing a cancer and/or preventing cancer relapsing such as substances used in conventional chemotherapy, targeted therapy, epigenetic therapy and radiation therapy e.g. for example a co-agent can be selected from Methotrexate, Raltitrexed, Pemetrexed, Mercaptopurine, Thioguanine, Pentostatine, Cladribine, Fludarabine, 5 fluoro-uracile, Tegafur uracile, Capecitabine, Cytarabine, Hydroxycarbamide, Gemcitabine, Chlorambucil, Melphalan, Chlormethine, Estramustine, Ifosfamide, Cyclophosphamide, Fotemustine, Lomustine, Carmustine, Streptozocine, Organoplatine, Carboplatine, Cisplatin, Oxaliplatin, Thiotepa, Altretamine, Procarbazine, Temozolomide, Dacarbazine, Busulfan, Mitomycine C, Pipobroman, Irinotecan, Topotecan, Anthracycline, Epirubicine, Daunorubicine, Doxorubicine, Pirarubicine, Idarubicine, Mitoxantrone, Amsacrine, Elliptinium, Actinomycine D, Etoposide, Bleomycine, Vinorelbine, Vindesine, Vincristine, Vinblastine, Paclitaxel, Docetaxel, Dasatinib, Erlotinib, Imatinib, Sorafenib, Sunitinib, Trastuzumab, Alemtuzumab, Rituximab, Bevacizumab, Cetuximab, Panitumumab and Blinatumomab.

The present invention also relates to a method for the preparation of a compound of formula (I) or a pharmaceutically acceptable salt and/or solvate thereof as defined above comprising the following steps:
(i) reacting a compound of following formula (II): 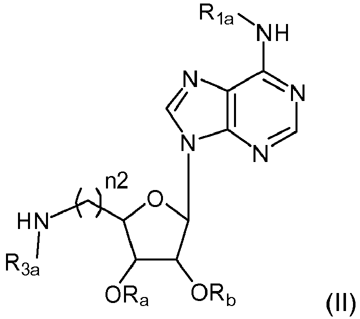 in which n2 is as defined above, R₁ₐ represents a R₁ group as defined above optionally in a protected form, R₃ₐ represents a R₃ group as defined above optionally in a protected form, and Rₐ and R_{b} represent, independently of one another, H or a O-protecting group,
   with a compound of following formula (III): 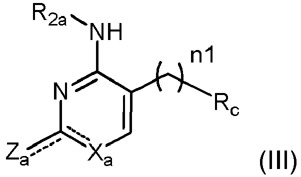 in which n1 is as defined above, R₂ₐ represents a R₂ group as defined above optionally in a protected form, Zₐ represents a Z group as defined above optionally in a protected form, Xₐ represents a X group as defined above optionally in a protected form, and R_{c} represents a leaving group, in order to obtain a compound of following formula (I-1): 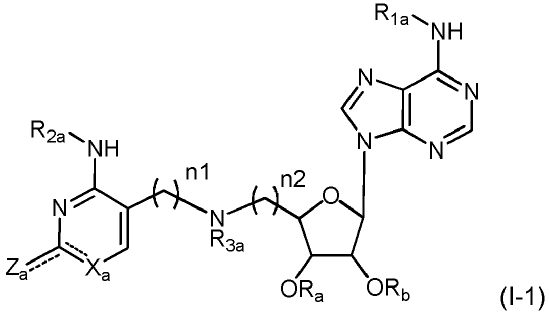 in which n1, n2, Xₐ, Zₐ, R₁ₐ, R₂ₐ, R₃ₐ, Rₐ and R_{b} are as defined above, which is a compound of formula (I) optionally in a protected form,
(ii) when the compound of formula (I-1) is a compound of formula (I) in a protected form, deprotecting the compound of formula (I-1) in order to obtain a compound of formula (I) (in an unprotected form), and
(iii) optionally salifying and/or solvating the compound of formula (I) obtained previously in order to obtain a pharmaceutically acceptable salt and/or solvate of compound of formula (I).

### Step (i):

The term "protected form", as used in the present invention, refers to a form of a multifunctional compound, in which a reactive site is blocked by a chemical group, called a protecting group, so as to allow selectively performing a chemical reaction on another unprotected reactive site. When the reactive site is an NH or OH group, the protecting group is named an O-protecting group or an N-protecting group respectively.

The term "O-protecting group" as used in the present invention refers to a substituent that protects hydroxyl groups (OH) against undesirable reactions during synthetic procedures such as those O-protecting groups disclosed in Greene's Protective Groups In Organic Synthesis, 4th edition, 2007, John Wiley & Sons, Hoboken, New Jersey. A hydroxyl group protected by a O-protecting group can be for example an ether, an ester, a carbonate, an acetal and the like. In particular, O-protecting groups can be a (C₁-C₆)alkyl optionally substituted with one or several (notably 1 to 3) halogen atoms (such as chlorine atoms), such as methyl, ethyl, *tert*-butyl or 2,2,2-trichloroethyl; an aryl-(C₁-C₆)alkyl, such as a benzyl, the aryl moiety being optionally substituted with one or several methoxy groups, such as benzyl (Bn) or *p*-methoxybenzyl (PMB); a trityl derivative of formula -CAr₁Ar₂Ar₃ such as triphenylmethyl (also called trityl - Tr), (4-methoxyphenyl)diphenylmethyl (also called methoxytrityl - NMT) or bis-(4-methoxyphenyl)phenylmethyl (also called dimethoxytrityl - DMT); a substituted methyl group of formula -CH₂OR_{GP2} or -CH₂SR_{GP2} (in particular -CH₂OR_{GP}), for example, methoxymethyl (MOM), benzyloxymethyl, 2-methoxyethoxymethyl (MEM), 2-(trimethylsilyl)ethoxymethyl or methylthiomethyl; a substituted ethyl group of formula -CH₂CH₂OR_{GP2} or-CH₂CH₂SR_{GP2} (in particular -CH₂CH₂OR_{GP2}), for example, ethoxyethyl (EE); a silyl group of formula -SiR_{GP3}R_{GP4}R_{GP5}, for example, trimethylsilyl (TMS), triethylsilyl (TES), *t*-butyldimethylsilyl (TBS or TBDMS) and *t*-butyldiphenylsilyl (TBDPS); a carbonylated group of formula -CO-R_{GP6} such as acetyl (Ac), pivaloyl (Piv or Pv) or benzoyl (Bz) or of formula -CO₂-R_{GP7} such as allyloxycarbonyl (Alloc) or 9-fluorenylmethyloxycarbonyl (Fmoc); or a tetrahydropyranyl ( 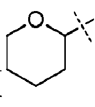 ) (THP) or tetrahydrofuranyl ( 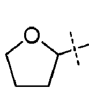 ) group; with Ar₁, Ar₂ and Ar₃ representing, independently from one another, an aryl, such as a phenyl, optionally substituted with one or several methoxy groups; R_{GP2} representing a (C₁-C₆)alkyl (such as methyl or ethyl) optionally substituted with an aryl (such as phenyl), a (C₁-C₆)alkoxy (such as methoxy) or a trialkylsilyl group (such as SiMe₃); R_{GP3}, R_{GP4} and R_{GP5} representing, independently from one another, a (C₁-C₆)alkyl or aryl (such as phenyl) group; and R_{GP6} and R_{GP7} representing, independently of each other, a (C₁-C₆)alkyl, a (C₂-C₆)alkenyl, an aryl, an aryl-(C₁-C₆)alkyl or a 9-fluorenylmethyl group.
In the case of the protection of a COOH group, the O-portecting group can be a (C₁-C₆)alkyl such as a *tert*-butyl.
Rₐ and R_{b} can form together a (C₁-C₆)alkanediyl group, such as an isopropylidene group, so as to form a cyclic acetal.

The term "N-protecting group", as used in the present invention, refers to those groups intended to protect an amine function (notably a primary amine function) against undesirable reactions during synthetic procedures. Commonly used N-protecting groups are disclosed in Greene's Protective Groups In Organic Synthesis, 4th edition, 2007, John Wiley & Sons, Hoboken, New Jersey. An amine function protected by a N-protecting group can be a carbamate, an amide, a sulfonamide, an N-alkyl derivative, an amino acetal derivative, a N-benzyl derivative, an imine derivative, an enamine derivative or a N-heteroatom derivative. In particular, N-protecting groups can be formyl; an aryl, such as a phenyl, optionally substituted with one or several methoxy groups such as p-methoxyphenyl (PMP); an aryl-(C₁-C₆)alkyl, such as a benzyl, the aryl moiety being optionally substituted with one or several methoxy groups, such as benzyl (Bn), *p*-methoxybenzyl (PMB) or 3,4-dimethoxybenzyl (DMPM); -CO-R_{GP1} such as acetyl (Ac), pivaloyl (Piv or Pv), benzoyl (Bz) or p-methoxybenzylcarbonyl (Moz); -CO₂-R_{GP1} such as *t*butyloxycarbonyl (Boc), trichloroethoxycarbonyl (TROC), allyloxycarbonyl (Alloc), benzyloxycarbonyl (Cbz or Z) or 9-fluorenylmethyloxycarbonyl (Fmoc); -SO₂-R_{GP1} such as phenylsulfonyl, tosyl (Ts or Tos) or 2-nitrobenzenesulfonyl (also called nosyl - Nos or Ns); and the like, with R_{GP1} representing a (C₁-C₆)alkyl optionally substituted with one or several halogen atoms such as F or Cl; a (C₂-C₆)alkenyl such as an allyl; an aryl, such as a phenyl, optionally substituted with one or several groups chosen among OMe (methoxy) and NO₂ (nitro); an aryl-(C₁-C₆)alkyl, such as a benzyl, the aryl moiety being optionally substituted with one or several methoxy groups; or a 9-fluorenylmethyl group.
In particular, the N-protecting group can be a -CO₂-R_{GP1} or -SO₂-R_{GP1} group such as a Nos or Boc group.

The term "leaving group" as used in the present invention refers to a chemical group which can be easily replaced with a nucleophile during a nucleophile substitution reaction, the nucleophile bearing an NH group as nucleophile group in the present case. Such a leaving group can be in particular a halogen atom or a sulfonate. The sulfonate is in particular a group -OSO₂-R_{LG} with R_{LG} representing a (C₁-C₆)alkyl, aryl, aryl-(C₁-C₆)alkyl or (C₁-C₆)alkyl-aryl group, the said group being optionally substituted with one or several halogen atoms such as fluorine atoms. The sulfonate can be notably a mesylate (CH₃-S(O₂)O-), a triflate (CF₃-S(O)₂O-) or a tosylate (p-Me-C₆H₄-S(O)₂O-).
The leaving group will be in particular a halogen atom (e.g. Br, Cl or I) such as a bromine.

The reaction of step (i) advantageously is performed in the presence of a base such as K₂CO₃, TEA or DIPEA.

The reaction can be performed in a solvent such as dimethylformamide (DMF) or acetonitrile.

The reaction is carried out for example at room temperature, i.e. more particularly at a temperature comprised between 15 and 40°C, preferably between 20 and 30 °C and notably of about 25°C.

According to a particular embodiment, R₁ₐ represents a R₁ group (unprotected form) and R₂ₐ represents a R₂ group (unprotected form).

According to another particular embodiment, Rₐ and R_{b} each represent a O-protecting group.

According to a preferred embodiment, R₁ₐ represents a R₁ group (unprotected form), R₂ₐ represents a R₂ group (unprotected form) and Rₐ and R_{b} each represent a O-protecting group. Rₐ and R_{b} can form together a (C₁-C₆)alkanediyl group, such as an isopropylidene group, so as to form a cyclic acetal.

### Step (ii):

Deprotection step(s) can be carried out in order to obtain an unprotected form of the compound of formula (I) according to the invention. Such steps and their reaction conditions are well known to the one skilled in the art and are described notably in Greene's Protective Groups In Organic Synthesis, 4th edition, 2007, John Wiley & Sons, Hoboken, New Jersey, as well as in the experimental part below.

### Step (iii):

The salification and/or solvatation step can be carried out by methods well known to the one skilled in the art, in particular by reaction of the compound of formula (I) with a pharmaceutically acceptable acid (organic or inorganic acid) or base (organic or inorganic base) and/or solvent, as defined previously.

The solvent can be notably the solvent used in the last step of the preparation of the compound according to the invention.

The compound obtained can be separated from the reaction medium by methods well known to the person skilled in the art, such as by extraction, by evaporation of the solvent or by precipitation or crystallisation (followed by filtration).

The compound can be also purified if necessary by methods well known to the person skilled in the art, such as by recrystallisation, by distillation, by chromatography on a column of silica gel or by high performance liquid chromatography (HPLC).

The present invention is illustrated by the following non-limiting examples.

### EXAMPLES

The following abbreviations have been used in the following examples:
- AIBN: : Azobisisobutyronitrile
- Boc: : *tert*-Butyloxycarbonyl
- BSA: : *N*,*O*-Bis(trimethylsilyl)acetamide
- cat.: : catalytic
- DCC: : *N*,*N*'-Dicyclohexylcarbodiimide
- DCM: : Dichloromethane
- DIPEA: : *N*,*N*-Diisopropylethylamine
- DMAP: : 4-Dimethylaminopyridine
- DMF: : Dimethylformamide
- DMSO: : Dimethylsulfoxide
- DTT: : Dithiothreitol (Cleland's reagent)
- ESI: : Electrospray ionisation
- HPLC: : High Performance Liquid Chromatography
- HRMS: : High Resolution Mass Spectrometry
- HSA: : Human serum albumin
- MOM: : Methoxymethyl
- NBS: : *N*-Bromosuccinimide
- NMR: : Nuclear Magnetic Resonance
- Nos: : 2-Nitrobenzenesulfonyl / Nosyl
- RT: : Room temperature
- SAH: : S-Adenosyl-L-homocysteine
- SAM: : S-Adenosyl-L-methionine
- TEA: : Triethylamine
- TFA: : Trifluoroacetic acid
- TLC: : Thin Layer Chromatography
- TMS: : Trimethylsilyl
- Tos: : 4-Methylbenzenesulfonyl / Tosyl

### I. Synthesis of the compounds according to the invention

### 1. Starting material

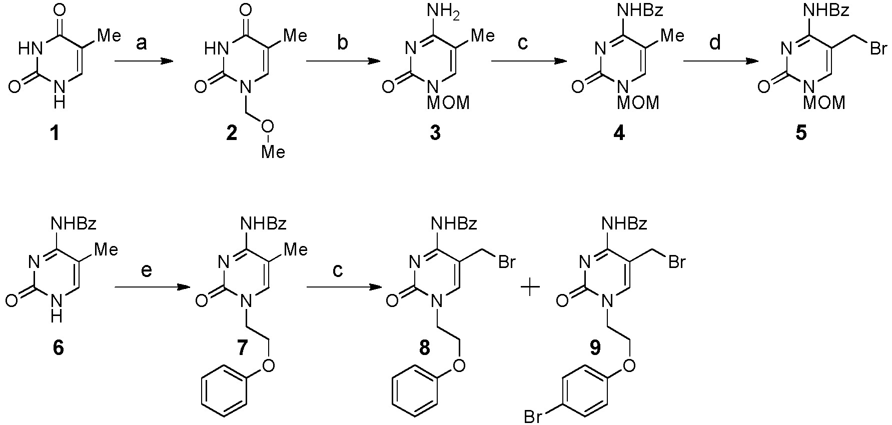

***Scheme 1***. Synthesis of cytosine analogs **5** and **8.** a) MOMCI, BSA, DCM, RT, 18h, 81%. b) (*i*)TosCl, Me-piperidine, TEA, MeCN, 2h, RT; (*ii*) NH₄OH, RT, 2h. 90% c) Bz₂O, DMAP, TEA, DMF, RT, overnight, 64%. d) NBS, AIBN, benzene, 70°C, 2h. e) PhO(CH₂)₂Br, BSA, DCM, RT, 18h, 74%.

To synthesize all compounds, a convergent synthetic pathway was developed starting from the protected bromomethylcytosine derivatives **5** or **8** and by-product **9.**

The key synthons **5, 8** and **9** were freshly prepared from the protected 5-methylcytosine derivatives **4** and **7. 4** was obtained in 3 steps with 47% yield from thymine **7** by *N*-alkylation with a MOM group, conversion into 5-methylcytosine and protection of the exocyclic amine with benzoyl group. **7** was obtained by *N*-alkylation with phenoxyethyl group of the protected cytosine synthesized as described in Buchini et *al.* (2006). Radical bromination in the benzylic position of **4** and **7** was achieved with NBS and AIBN leading to the desired unstable bromides **5** and **8** (and **9** as by-product), respectively, that were used immediately without purification.

### 1.1. N1-(methoxymethyl)-thymine (2)

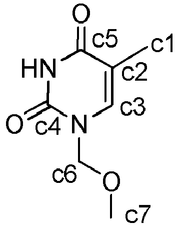

To a solution of thymine **1** (1 g; 7.94 mmol) in 250 mL of DCM was added BSA (4.8 mL; 19.4 mmol). The mixture was stirred 1 h at RT. To the reaction mixture was added chloromethylmethylester (784 µL; 10.32 mmol) and the mixture was stirred 17h at RT. The solvent was removed and the residue was purified by silica gel chromatography using the eluent cyclohexane / ethyl acetate (7/3) to give **2** (1.088 g; 6.4 mmol; 81 %) as a white powder.
**¹H NMR (500MHz ; CDCl₃) δ** 9.30 (s, 1 H, HNH), 7.15 (m, 1 H, Hc3), 5.12 (s, 2H, Hc6), 3.41 (s, 3H, Hc7), 1.98 (s, 3H, Hc1).
**¹³C NMR (125MHz, CDCl₃) δ** 164.13 (Cc5), 151.32 (Cc4), 138.81 (Cc3), 111.73 (Cc2), 77.64 (Cc6), 56.83 (Cc7), 12.32 (Cc1).
**HRMS-ESI(m/z)** calculated for C₇H₁₀N₂Na₁O₃ [M+Na]⁺: 193.0584 ; Found: 193.0566.

### 1.2. N1-(methoxymethyl)-5-methyl-cytosine (3)

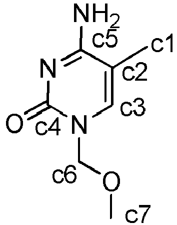

Tosyl chloride (1.58 g; 8.3 mmol) was added to a solution of **2** (680 mg; 4 mmol), N-methylpiperidine (560 µL, 4.68 mmol) and TEA (1.16 mL; 8.3 mmol) in 6 mL of acetonitrile at 0 °C. The mixture was stirred 2h at RT. Then, NH₄OH was added to the reaction mixture and the mixture was stirred 2h at RT. The solvent was removed and the residue was purified by silica gel flash chromatography using a linear gradient of DCM / methanol (0 to 20%, MeOH) to give **3** (610 mg; 3.61 mmol; 90%) as a white solid.
**¹H NMR (500MHz ; CDCl₃) δ** 7.44 (s, 1 H, Hc3), 7.34 (s, 1 H, HNH2), 6.84 (s, 1 H, HNH2), 4.97 (s, 2H, Hc6), 3.21 (s, 3H, Hc7), 1.82 (s, 3H, Hc1).
**¹³C NMR (125MHz, CDCl₃) δ** 166.37 (Cc5), 156.30 (Cc4), 142.97 (Cc3), 101.67 (Cc2), 78.59 (Cc6), 56.21 (Cc7), 13.34 (Cc1).
**HRMS-ESI(m/z)** calculated for C₇H₁₁N₃Na₁O₂ [M+Na]⁺: 192.0743 ; Found: 192.0733.

### 1.3. N1-(methoxvmethyl)-N⁴-benzoyl-5-methyl-cytosine (4)

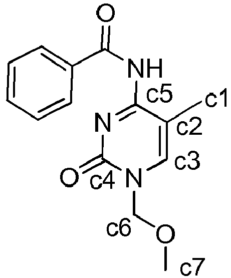

To a solution of **3** (159 mg; 0.94 mmol) in 2 mL of dimethylformamide was added benzoic anhydride (255 mg; 1.13 mmol), TEA (260 µL; 1.88 mmol) and a catalytic quantity of DMAP. The mixture was stirred at RT overnight. The reaction mixture was diluted by ethyl acetate and the organic phase was washed with water and brine then dried over sodium sulphate. The solvent was removed and the crude product was purified by silica gel flash chromatography using a linear gradient of DCM / ammonia solution 0.5N in methanol (0→10%, MeOH/NH₃) to give **4** as a white amorphous solid (163 mg; 0.60 mmol; 64%).
**¹H NMR (500MHz** ; **CDCl₃) δ** 13.21 (s, 1 H, HNH), 8.31 (m, 2H, HBz), 7.52 (m, 1 H, HBz), 7.44 (m, 2H, HBz), 7.25 (q, 1 H, *J=* 1.2 Hz, Hc3), 5.11 (s, 2H, Hc6), 3.40 (s, 3H, Hc7), 2.11 (d, 3H, *J*= 1.2 Hz, Hc1).
**¹³C NMR (125MHz, CDCl₃) δ** 179.8 (CBz), 160.0 (Cc5), 148.9 (Cc4), 139.8 (Cc3), 137.1 (CBz), 132.6 (CBz), 130.0 (CBz), 128.2 (CBz), 112.6 (Cc2), 78.2 (Cc6), 57.1 (Cc7), 13.5 (Cc1).
**HRMS-ESI(m/z)** calculated for C₇H₁₁N₃Na₁O₂ [M+Na]⁺: 296.1006 ; Found: 296.0991.

### 1.4. N1-(methoxymethyl)-N⁴-benzoyl-5-bromomethyl-cytosine (5)

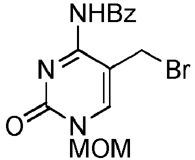

To a solution of **4** (100 mg; 360 µmol) in dry benzene (5 mL) under argon atmosphere were added NBS (125 mg, 720 µmol) and AIBN (17 mg; 0.1 mmol) and the mixture was heated at 70°C for 1 h. The reaction was monitored by TLC to prevent by-product production (cyclohexane / ethyl acetate 7:3). After cooling, the reaction mixture was diluted with 10 mL of the organic phase was washed with 1 M NaHCO₃ and brine, dried on magnesium sulphate and the solvent was removed by vacuum. The crude product **5** was used immediately without further purification (125 mg; 0.35 mmol; 98%).

### 1.5. N1-(2-phenoxyethyl)-N⁴-benzoyl-5-methyl-cytosine (7)

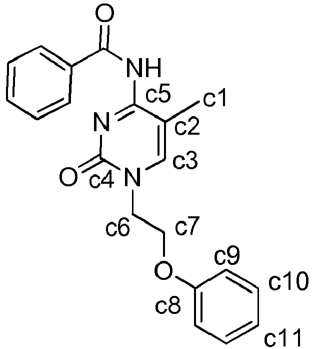

To a solution of *N*-benzoyl-5-methylcytosine synthesized from Buchini *et al.* (2006) (500 mg, 2.18 mmol) in 70 mL of DCM was added BSA (1.3 mL; 5.4 mmol). The mixture was stirred 1h at RT. To the reaction mixture was added chloromethylmethylester (218 µL; 2.87 mmol) and the mixture was stirred 17h at RT. The solvent was removed and the residue was purified by silica gel chromatography using the eluent cyclohexane / ethyl acetate (7/3) to give **7** (561 mg; 1.61 mmol; 74%) as a white powder.
**¹H NMR (500MHz ; CDCl₃) δ** 13.44 (s, 1 H, HNH), 8.35 (d, J=7.2Hz, 2H, HBz), 7.52 (ddd, J=2.0, 6.0, 7.3Hz, 1 H, HBz), 7.48-7.44 (m, 2H, HBz), 7.39 (d, 1 H, *J=* 0.9 Hz, Hc3), 7.35-7.29 (m, 2H, Hc10), 7.05-6.98 (m, 1 H, Hc11), 6.92-6.87 (m, 2H, Hc9), 4.28 (t, J=4.6Hz, 2H, Hc7), 3.40 (t, J=4.6Hz, 2H, Hc6), 2.14 (d, 3H, *J*= 1.1 Hz, Hc1).
**¹³C NMR (125MHz, CDCl₃) δ** 179.6 (CBz), 160.3 (Cc5), 157.8 (Cc8), 148.5 (Cc4), 142.7 (Cc3), 137.2 (CBz), 132.4 (CBz), 129.9 (CBz), 129.7 (Cc10), 128.1 (CBz), 121.6 (Cc11), 114.4 (Cc9), 111.2 (Cc2), 65.5 (Cc6), 48.8 (Cc7), 13.3 (Cc1).
**HRMS-ESI(m/z)** calculated for C₂₀H₂₀N₃O₃ [M+H]⁺: 350.1499 ; Found: 350.1491.

### 1.6. N1-(2-phenoxyethyl)-N⁴-benzoyl-5-bromomethyl-cytosine (8) and N1-(2-para-bromophenoxyethyl)-N⁴-benzoyl-5-bromomethyl-cytosine (9)

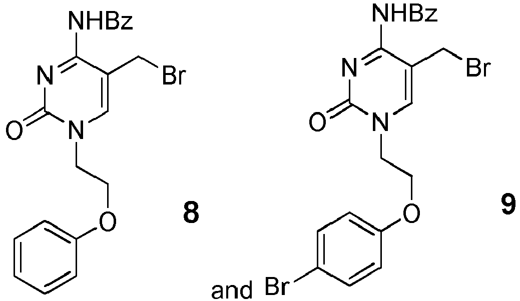

To a solution of **7** (126 mg; 360 µmol) in dry benzene (5 mL) under argon atmosphere were added NBS (125 mg, 720 µmol) and AIBN (17 mg; 0.1 mmol) and the mixture was heated at 70°C for 1 h. The reaction was monitored by TLC to prevent by-product production (cyclohexane / ethyl acetate 7:3). After cooling, the solvent was evaporated. The residue was diluted with 10 mL of DCM and the organic phase was washed with 1 M NaHCO₃ and brine, dried on magnesium sulphate and the solvent was removed by vacuum. The crude mixture of **8** and **9** was used immediately without further purification (119 mg; 277 µmol; 77%).

### 2. Compound A

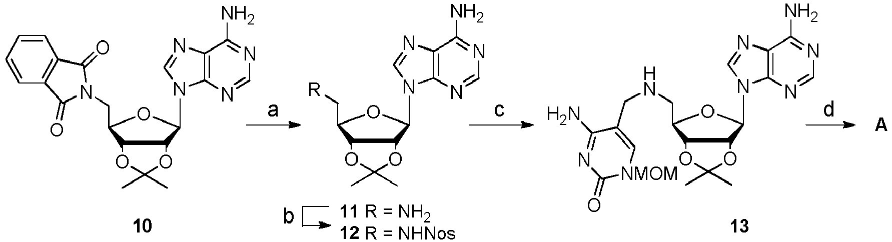

**Scheme 2.** Synthesis of compound **A.** a) CH₃NHNH₂, ethanol, RT, 18h; b) NosCl, TEA, DMF, RT, 6h. c) *i)* **5,** DMF, K₂CO₃, RT, 1 h. *ii*) NH₃/MeOH. *iii*) PhSH, K₂CO₃, DMF, RT, 18h. d) TFA, water, RT, 1 h.

Compound **A** was synthesized starting from phthalimide **12** previously described (Mai and Comstock (2011)) (Scheme 2). Phthalimide removal upon addition of *N*-methylhydrazine followed by protection of the resulting amine **11** with nitrophenylsulfonyl chloride afforded nosylated 5'-deoxy-5'-amino-2',3'-isopropylidene-adenosine **12.** Substitution of bromide of bromomethylcytosine derivative **5** with sulfonamide **12** in the presence of K₂CO₃ was followed by treatment with ammonia in methanol to remove the benzoyl group of the cytosine moiety and then with thiophenol to remove the nosyl protective group. Compound **A** was obtained after cleavage of the isopropylidene group protecting the ribose group upon TFA/water treatment.

### 2.1. 2',3'-O-isopropylidene- 5'-deoxy-5'-aminoadenosine (11)

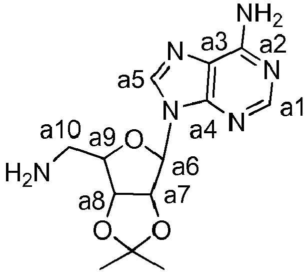

*N*-methylhydrazine 200 µL was added to a solution of **10** (500 mg; 1.15 mmol) in ethanol (2 mL). The mixture was stirred overnight at RT. The solvent was removed under reduce pressure and the residue was purified by silica gel flash chromatography using a linear gradient of ammonia 1 N in methanol (0→10% MeOH/NH₃) in DCM to afford **11** as a white solid (275 mg; 90 µmol; 78%)
**¹H NMR (500MHz; DMSO) δ** 8.36 (s, 1H, Ha1), 8.16 (s, 1H, Ha5), 7.35 (brs, 2H, HNH2), 6.08 (d, *J*=3.2Hz, 1 H, Ha6), 5.46 (dd, *J*=3.2, 6.4Hz, 1 H, Ha7), 4.98 (dd, *J*=2.7, 6.3Hz, 1 H, Ha8), 4.09 (ddd, *J*=2.9, 5.7, 11.4Hz, 1 H, Ha9), 2.71 (dd, *J*=6.0, 13.6 Hz, 1 H, Ha10A), 2.67 (dd, *J*=6.0, 13.6 Hz, 1 H, Ha10B), 1.51 (s, 3H, HMe), 1.27 (s, 3H, HMe).
**¹³C NMR (125MHz; DMSO) δ** 156.6 (Ca2), 153.2 (Ca1), 149.4 (Ca4), 140.4 (Ca5), 119.6 (Ca3), 113.5 (Cipr), 89.5 (Ca6), 87.4 (Ca9), 83.0 (Ca7), 82.0 (Ca8), 44.1 (Ca10), 27.5 (CMe), 25.7 (CMe).
**HRMS-ESI(m/z)** calculated C₁₃H₁₉N₆O₃ [M+H] :307.1513 ; Found: 307.1515.

### 2.2. 2',3'-O-isopropylidene- 5'-deoxy-5'-(2-nitrobenzenesulfonamido)adenosine (12)

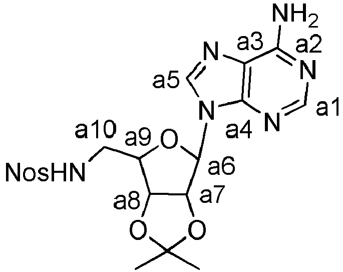

To a solution of **11**(150 mg; 49 µmol) in DMF (2 mL) and TEA (0.5 mL) was added 2-nitrophenylsufonyl chloride (270 mg; 1.23 mmol). The mixture was stirred for 6h at RT. The solvent was removed by vacuum and the residue was purified by silica gel flash chromatography using a linear gradient of ammonia 1N in methanol (0→10% MeOH/NH₃) in DCM to give **12** as white amorphous solid (200 mg; 41 µmol; 83%).
**¹H NMR (500MHz; DMSO) δ** 8.64 (brs, 1H, HNH), 8.29 (s, 1H, Ha1), 8.12 (s, 1H, Ha5), 7.92 (dd, *J*=1.2, 8.0Hz, 1H, HNos), 7.84 (dd, *J*=1.2, 8.0Hz, 1H, HNos), 7.79 (ddd, *J*=1.2, 6.4, 7.8Hz, 1H, HNos), 7.71 (ddd, *J*=1.2, 6.4, 7.7Hz, 1H, HNos), 7.41 (brs, 2H, HNH2), 6.10 (d, *J*=2.9Hz, 1 H, Ha6), 5.36 (dd, *J*=3.0, 6.3Hz, 1H, Ha7), 4.94 (dd, *J*=2.9, 6.3Hz, 1H, Ha8), 4.22 (ddd, *J*=2.9, 5.7, 11.5Hz, 1H, Ha9), 3.29 (dd, *J*=5.8, 13.6 Hz, 1H, Ha10A), 3.21 (dd, *J*=5.8, 13.6 Hz, 1 H, Ha10B), 1.51 (s, 3H, HMe), 1.27 (s, 3H, HMe).
**¹³C NMR (125MHz; DMSO) δ** 156.7 (Ca2), 153.0 (Ca1), 148.8 (Ca4), 148.0 (CNos), 140.6 (Ca5), 132.4 (CNos), 129.8 (Ccos), 124.8 (CNos), 119.7 (Ca3), 113.9 (Cipr), 89.9 (Ca6), 85.7 (Ca9), 83.2 (Ca7), 82.0 (Ca8), 45.1 (Ca10), 27.4 (CMe), 25.5 (CMe).
**HRMS-ESI(m/z)** calculated for C₃₃H₄₆N₇O₈ [M+H]⁺:492.1296 ; Found:492.1290.

### 2.3. 1-(methoxymethyl)-5-((2',3'-O-isopropylidene-5'-deoxyadenosin-5'-yl) aminomethyl)cytosine (13)

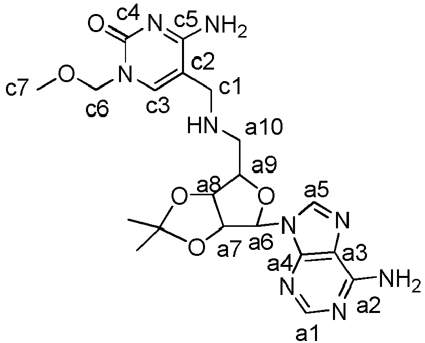

To a solution of **12** (50 mg; 102 µmol) in anhydrous DMF (2 mL) was added K₂CO₃ (41 mg; 306 µmol) and freshly prepared **5** (200 µmol). The mixture was stirred for 1 h at RT. The mixture was diluted with ethyl acetate and washed with water. The organic phase was washed with brine and dried with magnesium sulphate. Solvent was removed and the residue was dissolved in 7N ammonia in methanol. The mixture was stirred at RT for 6h. The solvent was removed under reduce pressure. The residue was dissolved in a suspension of K₂CO₃ (41 mg; 306 µmol) in DMF (1 mL). Thiophenol (25 µL; 250 µmol) was added and the mixture was stirred at RT overnight. The mixture was filtrated and the solvent was removed. The residue was purified by reversed phase HPLC using a linear acetonitrile gradient (0→60% CH₃CN) to obtain **13** as colourless foam (10 mg; 21 µmol; 21 %).
**¹H NMR (500MHz; DMSO+D₂O) δ** 8.33 (s, 1 H, Ha5), 8.08 (s, 1 H, Ha1), 7.49 (s, 1 H, Hc3), 6.06 (d, *J*=3.3Hz, 1 H, Ha6), 5.44 (dd, *J*=3.3, 6.3Hz, 1 H, Ha9), 4.97 (m, 3H, Ha7 and Hc7), 4.23 (m, 1 H, Ha8), 3.40 (d, *J*=6.1 Hz, 2H, Hc1), 3.20 (s, 3H, Hc7), 2.69 (m, 2H, Ha10), 1.54 (s, 3H, Hipr), 1.33 (s, 3H, Hipr).
**¹³C NMR (125MHz; DMSO+ D₂O) δ** 166.0 (Cc5), 156.4 (Ca2), 155.5 (Cc4), 153.1 (Ca1), 149.2 (Ca4), 143.9 (Cc3), 140.6 (Ca5), 119.4 (Ca3), 113.9 (Cipr), 104.1 (Cc2), 89.5 (Ca6), 83.0 (Ca9), 84.5 (Ca8), 82.6 (Ca7), 78.8 (Hc6), 56.3 (Cc7), 50.2 (Ca10), 46.9 (Cc1), 27.4 (Cipr), ), 25.7 (Cipr).
**HRMS-ESI(m/z)** calculated for C₂₀H₂₈N₉O₅ [M+H]⁺ : 474.2208 ; Found: 474.2202.

### 2.4. 1-(methoxymethyl)-5-(N-(5'-deoxyadenosin-5'-yl)aminomethyl)cytosine 5'-deoxy-5'-((1-(methoxymethyl)cytosin-5-yl)methylamino)adenosine(A)

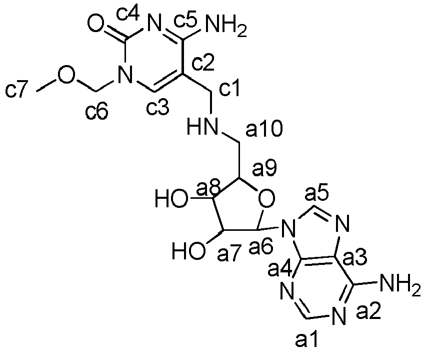

Water (50 µL) was added to a solution of **13** (10 mg; 21 µmol) in TFA (0.5 mL). The reaction mixture was stirred at RT for 1 h. The solvent was removed and the residue was co-evaporated three times with 1 N ammonia in methanol under reduce pressure to obtain **A** as colourless foam (8.1 mg; 19 µmol; 89%).
**¹H NMR (500MHz; D₂O) δ** 8.26 (s, 1H, Ha1), 8.12 (s, 1H, Ha5), 7.83 (s, 1H, Hc3), 6.10 (d, *J*=4.6Hz, 1 H, Ha6), 5.07 and 5.01 (d, *J*=10.6Hz, 2H, Hc6), 4.83 (t, *J*=4.5Hz, 1 H, Ha7), 4.53 (t, *J*=5.2Hz, 1 H, Ha8), 4.49 (q, *J*=5.8Hz, 1 H, Ha9), 4.17 and 4.12 (d, *J*=14.6Hz, 2H, Ha10), 3.60 (d, *J*=6.0Hz, 2H, Hc1), 3.30 (s, 3H, Hc7).
**¹³C NMR (125MHz; D₂O) δ** 164.8 (Cc5), 157.1 (Cc4), 155.5 (Ca2), 152.3 (Ca1), 149.2 (Cc3), 148.2 (Ca4), 140.7 (Ca5), 119.0 (Ca3), 98.6 (Cc2), 89.3 (Ca6), 79.8 (Cc6), 79.3 (Ca9), 72.8 (Ca7), 71.3 (Ca8), 56.1 (Cc7), 48.0 (Cc1), 44.0 (Ca10).
**HRMS-ESI(m/z)** calculated for C₁₇H₂₃N₉NaO₅ [M+Na]⁺: 456.1714 ; Found: 456.1699.

### 3. Compound B

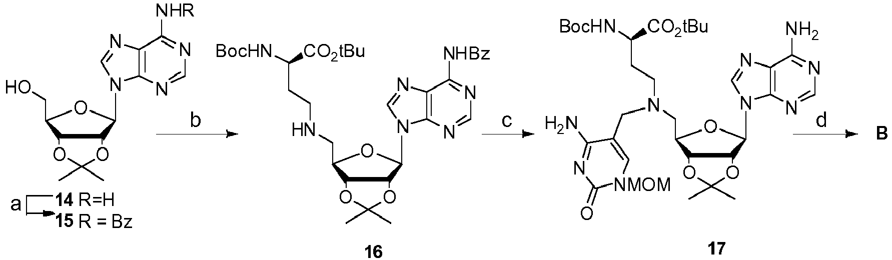

***Scheme 3.*** Synthesis of compound **B.** a) TMSCI, pyridine, RT, 0.5h then BzCl, RT, 3h, 90%. b) (*i*) TFA, DCC, pyridine, DMSO, RT, 2h; (*ii*) (S)-2-*tert-*butoxycarbonylamino-4-aminobutyric acid *tert*-butyl ester, MeOH, TEA, RT, 2h; *iii*) NaBH₃CN, MeOH, RT, 2h. 23% over 3 steps. c) (*i*) **5,** DMF, DIPEA, RT, 1 h; (*ii*) NH₃/MeOH, RT, 3h, 64% over 2 steps. d) TFA, water, RT, 1 h. 65%. Compound **B** was synthesized from the adenosine derivative **16** that was obtained from the protected adenosine **14** in 3 steps (23% overall yield), by standard oxidation with anhydrous DMSO and DCC using Moffatt oxidation conditions followed by reaction of the resulting adenosine-5'-carboxaldehyde with (S)-2-*tert*-butoxycarbonylamino-4-aminobutyric acid *tert*-butyl ester and reductive amination in the presence of NaBH₃CN. Substitution of the bromide atom of compound **5** with amine **16** in the presence of K₂CO₃ followed by treatment with ammonia in methanol first and then with TFA in water afforded the desired compound **B.**

### 3.1. N⁶-benzoyl-(2',3'-O-isopropylidene-5'-deoxy-5'-iodo)-adenosine (15)

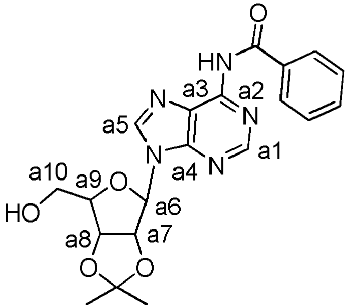

To a solution of **14** (307 mg, 1.0 mmol) in pyridine (5 mL) was added TMSCI (0.64 mL, 5 mmol).The mixture was stirred at RT for 30 min and benzoyl chloride(150 µL, 1.3 mmol) was added. The mixture was stirred at RT for 3 h and the mixture was cooled to 0 °C and diluted with water (1 mL). The mixture was stirred for 10 min and aqueous ammonia (2 mL) was added. The mixture was stirred for 30 minutes at RT and the solvents were removed. The residue was purified by silica gel flash chromatography using a linear gradient of DCM / methanol (0 to 10%, MeOH) to give **15** (276 mg; 0.90 mmol; 90%) as a white solid.
**¹H NMR (500MHz; DMSO) δ** 11.20 (s, 1H, HNH), 8.78 (s, 1H, Ha1), 8.68 (s, 1H, Ha5), 8.06 (d, J=7.3Hz, 2H, HBz), 7.65-7.55 (m, 3H, HBz), 6.29 (d, J=2.7Hz, 1H, Ha6), 5.45 (dd, J=2.7, 6.1Hz, 1H, Ha7), 5.12 (t, J=2.7Hz, HOH), 5.00 (dd, J=2.7, 6.3Hz, 1 H, Ha8), 4.28 (ddd, J=2.5, 6.2, 16.8Hz, 1 H, Ha9), 3.31 (dd, J=6.0, 13.6 Hz, 2H, Ha10), 1.57 (s, 3H, HMe), 1.35 (s, 3H, HMe).
**¹³C NMR (125MHz; DMSO) δ** 165.5 (CBz), 155.6 (Ca2), 153.3 (Ca1), 149.9 (Ca4), 141.5 (Ca5), 134.5 (CBz), 133.8 (CBz), 129.0 (CBz), 127.8 (CBz), 121.5 (Ca3), 115.5 (Cisop), 88.5 (Ca6), 87.5 (Ca9), 83.1 (Ca7), 82.1 (Ca8), 63.1 (Ca10), 27.0 (CMe), 25.4 (CMe).
**HRMS-ESI(m/z)** calculated for C₂₀H₂₂N₅O₅ [M+H] : 412.1615; Found: 412.1685.

### 3.2. tert-butyl 2-((Boc)amino)-4-(((2',3'-O-isopropylidene-N⁴-benzoyl-5'-deoxyadénosiny-5'-yl)amino)butanoate (16)

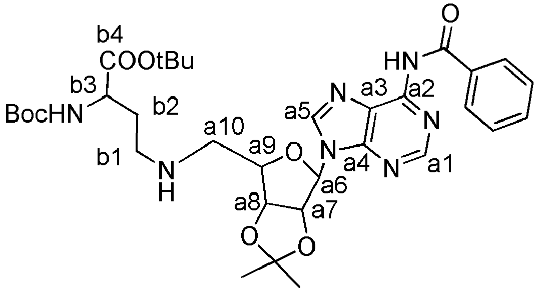

Protected adenosine **15** (370 mg; 900 µmol) and DCC (560 mg; 2.7 mmol) were dissolved in a mixture of 5 mL of DMSO and pyridine (90 µL, 90 µmol). TFA (110 µL; 450 µmol) was added and the reaction mixture was stirred at RT for 2h then filtrated to remove dicyclohexylurea. The solvents were removed by coevaporation with toluene (3x10 mL) under reduce pressure. The residue was used without further purification.

Crude aldehyde of protected adenosine and hydrochloride of (S)-2-tert-butoxycarbonylamino-4-aminobutyric acid tert-butyl ester (330 mg, 1.2 mmol) were dissolved in 10 mL of methanol and 100 µL of TEA. After stirring at RT for 2h, 100 µL of acetic acid and NaBH₃CN (100 mg, 1.6 mmol) were successively added and the reaction mixture was stirred at RT for 2 hours. The solvent was removed by vacuum and the residue was purified by silica gel chromatography using a linear gradient of methanol (0→10% MeOH) in DCM to afford **16** as a white amorphous solid (138 mg; 207 µmol; 23%).
**¹H NMR (500MHz; CDCl₃) δ** 8.83 (s, 1H, Ha5), 8.20 (s, 1H, Ha1), 8.03 (d, *J*=7.8Hz, 2H, HBz), 7.65 (m, 1H, HBz), 7.56 (t, *J*=7.8Hz, 1H, HBz), 6.01 (d, *J*=3.3Hz, 1 H, Ha6), 5.52 (m, 1 H, Ha7), 5.11 (m, 1 H, Ha8), 4.42 (m, 1 H, Ha9), 4.29 (m, 1 H, Ha9), 3.23 (s, 1 H, Hb3), 3.01 (m, 1 H, Ha10), 2.90 (m, 1 H, Ha10), 2.80 (m, 1 H, Hb1), 2.71 (m, 1 H, Hb1), 2.01 (m, 1 H, Hb2), 1.84 (m, 1 H, Hb2), 1.65 (m, 3H, HMe), 1.51-1.37 (m, 21 H, HMe and Ht-Bu and HBoc).
**¹³C NMR (125MHz; CCl₃) δ** 171.6 (Cb4), 164.5 (CBz), 155.7 (CBoc), 152.7 (Ca1), 151.3 (Ca2), 149.7 (Ca4), 142.3 (Ca5),133.5 (CBz), 132.9 (CBz), 128.9 (CBz), 127.9 (CBz), 123.7 (Ca3), 114.9 (Cᵢₚᵣ), 91.0 (Ca6), 85.4 (Ca9), 83.3 (Ca7), 82.0 (Ca8), 81.8 (CtBu), 79.6 (CBoc), 52.6 (Cb3), 51.1 (Ca10), 46.2 (Cb1), 32.2 (Cb2), 28.3 (CtBu), 28.04 (CBoc), 27.7 (Cb2), 27.2 (CMe), 25.5 (CMe).
**HRMS-ESI(m/z)** calculated for C₃₃H₄₆N₇O₈ [M+H] : 668.3402; Found: 668.3453.

### 3.3. tert-butyl 2-((Boc)amino)-4-((1-(methoxymethyl)cytosin-5-yl)methyl)(2'.3'-isopropylidene-5'-deoxyadenosin-5'-yl)amino)butanoate (17)

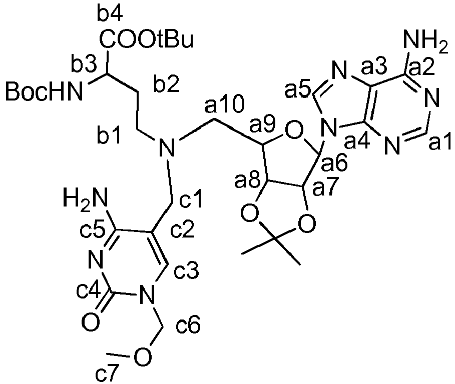

Freshly prepared **5** (125 mg; 0.35 mmol) was added to a solution of **16** (68 mg; 0.1 mmol) and in DMF (3 mL) and DIPEA (100 µL). After 1h at RT, the reaction mixture was diluted with ethyl acetate and the organic phase was washed with 1M NaHCO₃ and brine, dried on magnesium sulphate and the solvent was removed by vacuum. The residue was dissolved in 7N ammonia in methanol and stirred for 3h at RT. Then the solvent was removed and the residue was purified by silica gel flash chromatography using a linear gradient of ammonia 1N in methanol (0→10% MeOH/NH₃) in DCM then by reversed phase HPLC using a linear acetonitrile gradient with 0.2% of TEA (0→80% CH₃CN) to afford **17** as a white powder (47 mg; 64 µmol; 64%).
**¹H NMR (500MHz; DMSO) δ** 8.29 (s, 1 H, Ha5), 8.12 (s, 1 H, Ha1), 7.51 (s, 1 H, Hc3), 7.36 (m, 3H, HNH and HNH2), 7.10 (d, *J*=8.0Hz, 1 H, HNH), 6.73 (brs, 1 H, HNH), 6.18 (d, *J*=2.2Hz, 1H, Ha6), 5.46 (dd, *J*=2.2, 6.3HZ, 1H, Ha7), 5.0 (d, *J*=9.6Hz, 1 H, Hc6), 4.98 (dd, *J*=3.5, 6.3HZ, 1 H, Ha8), 4.89 (d, *J*=9.6Hz, 1 H, Hc6), 4.25 (m, 1 H, Ha9), 3.77 (m, 1 H, Hb3), 3.31 (m, 2H, Hc1), 3.21 (s, 3H, Hc7), 3.15 (m, 1 H, Hc1), 2.84 (m, 1 H, Hb1), 2.55 (m, 2H, Hb1 and Ha10), 2.32 (m, 1 H, Ha10), 1.71 (m, 2H, Hb2), 1.53 (s, 3H, HMe), 1.36 (s, 9H, Ht-Bu), 1.34 (s, 9H, Boc), 1.32 (s, 3H, HMe).
**¹³C NMR (125MHz; DMSO) δ** 172.2 (Cb4), 165.9 (Cc5), 156.6 (Ca2), 156.0 (Cc4), 155.9 (CBoc), 153.1 (Ca1), 149.0 (Ca4), 144.8 (Cc3), 140.6 (Ca5), 119.6 (Ca3), 114.0 (Cipr), 102.4 (Cc2), 89.0 (Ca6), 84.2 (Ca9), 83.7 (Ca7), 83.3 (Ca8), 80.7 (Ct-Bu), 78.8 (Cc6),78.5 (CBoc), 56.3 (Cc7), 55.6 (Cb1), 53.4 (Cc1), 53.0 (Cb3 ), 50.1 (Ca10), 28.6 (Ct-Bu), 28.0 (CBoc), 27.7 (Cb2), 27.4 (CMe), 25.8 (CMe).
**HRMS-ESI(m/z)** calculated for C₃₃H₅₁N₁₀O₉ [M+H] :731.3835; Found:731.3829.

### 3.4. 2-amino-4-(((1-(methoxymethyl)cytosin-5-yl)methyl)((5'-deoxy-5'-(adenosin-5'-yl)amino)butanoic acid (B)

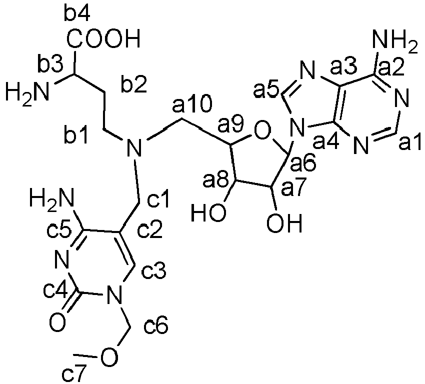

Water (100µL) was added to a solution of **17** (40 mg; 55 µmol) in TFA (1 mL). The reaction mixture was stirred at RT for 1 h. The solvent was removed and the residue was purified by reversed phase HPLC using a linear acetonitrile gradient (0→60% CH₃CN) to obtain **B** as a colourless foam powder (19 mg; 36 µmol; 65%).
**¹H NMR (500MHz; D₂O) δ** 8.10 (s, 1 H, Ha5), 8.08 (s, 1 H, Ha1), 7.51 (s, 1 H, Hc3), 5.90 (d, *J*=2.1 Hz, 1 H, Ha6), 4.84 (d, *J*=Hz, 1 H, Hc6), 4.79 (d, *J*=Hz, 1 H, Hc6), 4.67 (m, 1 H, Ha7), 4.25-4.17 (m, 2H, Ha8 and Ha9), 3.66-3.55 (m, 2H, Hb3 and Ha10), 3.47 (dd, *J*=13.6Hz, 1 H, Ha10), 3.15 (s, 3H, Hc7), 2.93 (m, 2H, Hb1), 2.78 (m, 2H, Hc1), 2.00 (m, 1 H, Hb2), 1.92 (m, 1 H, Hb2).
**¹³C NMR (125MHz; D₂O) δ** 173.8 (Cb4), 164.1 (Cc5), 154.7 (Ca2), 156.0 (Cc4), 151.6 (Ca1), 148.6 (Ca4), 146.3 (Cc3), 140.7 (Ca5), 118.9 (Ca3), 115.1 (Cipr), 117.4 (Cc2), 88.6 (Ca6), 80.9 (Ca9), 72.8 (Ca7), 71.9 (Ca8), 79.6 (Cc6), 56.1 (Cc7), 54.7 (Cb1), 53.4 (Cb3), 52.4 (Ca10), 50.7 (Cc1), 26.7 (Cb2).
**HRMS-ESI(m/z)** calculated for C₂₁H₃₁N₁₀O₇ [M+H] :535.2372; Found: 534.2376.

### 4. Compounds C and D

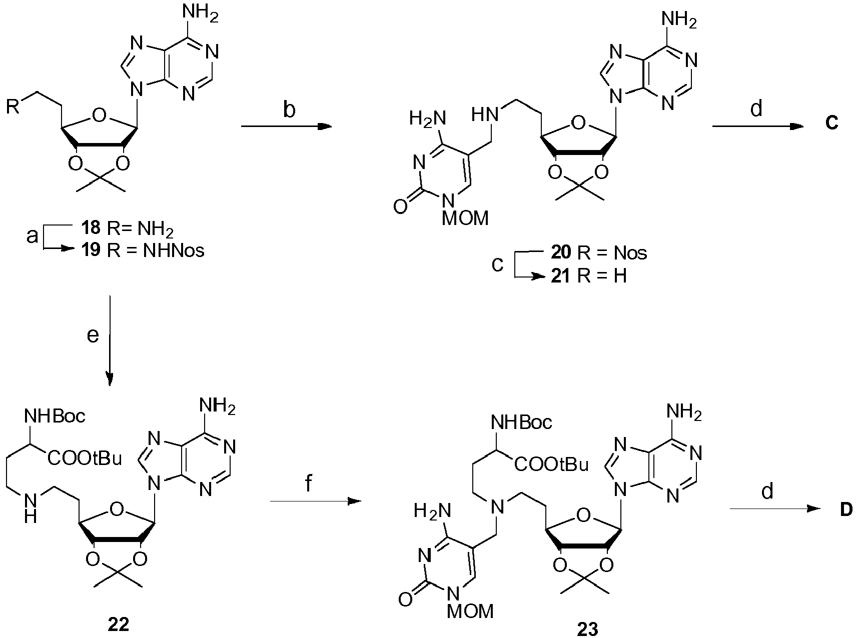

***Scheme 4.*** Synthesis of transition state analogs **C** and **D.** a) NosCl, DMAP cat., pyridine, RT, 6h, 77%. b) *i)* **5,** DMF, K₂CO₃, RT, 1h. *ii*) NH₃/MeOH, 46% over 2 steps. c) PhSH, K₂CO₃, DMF, RT, 18h, 77%. d) TFA, water, RT, 1 h, 89%. e) *i*) (*S*)-2-*tert*-butoxycarbonylamino-4-bromobutyric acid *tert*-butyl ester, MeOH, TEA, RT, 2h. *ii*) PhSH, K₂CO₃, DMF, RT, 18h, 53% over 2 steps. f) *i)* **5,** DMF, DIPEA, RT, 1h, 44%. *ii)* NH₃/MeOH, 87%.

Analogs **C** to **D** were obtained starting from the key synthon **19** that was produced by nosylation of 5'-aminomethyl-5'-deoxy-2',3'-*O-*isopropylideneadenosine **18** previously described in Lerner *et al. (2003). N-*alkylation of nosylated adenosine derivative **19** or secondary amine **22** by cytosine bromide derivative **5** followed by deprotection afforded compounds **C and D.**

### 4.1. 2',3'-O-isopropylidene- 5'-deoxy-5'-(2-nitrobenzenesulfonamidomethyl) adenosine (19)

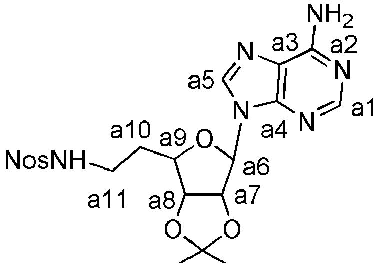

To a solution of **18** (1 g; 3.13 mmol) in pyridine (20 mL) was added 2-nitrophenylsufonyl chloride (826 mg; 3.73 mmol) portionwise. The mixture was stirred overnight at RT. The solvent was removed by vacuum and the residue was purified by silica gel flash chromatography using a linear gradient of ammonia 1 N in methanol (0→10% MeOH/NH₃) in DCM to give **19** as white amorphous solid (1.21 g; 2.40 mmol; 77%).
**¹H NMR (500MHz; CDCl₃)) δ** 8.29 (s, 1 H, Ha1), 8.08-8.05 (m, 1 H, HNos), 7.88 (s, 1H, Ha5), 7.82-7.77 (m, 1H, HNos), 7.72-69 (m, 2H, HNos), 6.01 (brt, J=5.4Hz, 1H, HNH2), 5.98 (d, *J*=2.4Hz, 1H, Ha6), 5.70 (brs, 1H, HNH), 5.46 (dd, *J*=2.6, 6.4Hz, 1 H, Ha7), 5.00 (dd, *J*=4.5, 6.4Hz, 1 H, Ha8), 4.24 (dt, *J*=4.5, 7.9Hz, 1H, Ha9), 3.31-3.18 (m, 1H, Ha11), 2.10-1.98 (m, 1H, Ha10), 1.61 (s, 3H, HMe), 1.38 (s, 3H, HMe).
**¹³C NMR (125MHz; CDCl₃) δ** 155.5 (Ca2), 153.2 (Ca1), 149.2 (Ca4), 147.8 (CNos), 140.2 (Ca5), 133.8 (CNos), 133.4 (CNos), 132.6 (CNos), 130.8 (CNos), 125.3 (CNos), 120.4 (Ca3), 114.9 (Cipr), 90.2 (Ca6), 84.8 (Ca9), 83.7 (Ca7), 83.5 (Ca8), 40.7 (Ca11), 45.1 (Ca10), 27.3 (CMe), 25.4 (CMe).
**HRMS-ESI(m/z)** calculated for C₂₀H₂₄N₇O₇S [M+H]⁺:506.1452 ; Found:506.1480.

### 4.2. 2',3'-O-isopropylidene-5'-deoxy-5'-(N-((1-(methoxymethyl)-cytosin-5-yl)methyl)-2-nitrophenylsulfonamido)methyl)adenosine (20)

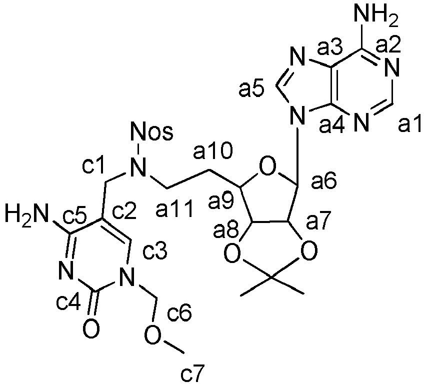

To a solution of derivative **19** (52 mg; 103 µmol) in anhydrous DMF (2 mL) was added K₂CO₃ (41 mg; 306 µmol) and freshly prepared **5** (200 µmol). The mixture was stirred for 4h at RT. The mixture was diluted with ethyl acetate and washed with water. The organic phase was washed with brine and dried with magnesium sulphate. Solvent was removed and the residue was dissolved in 7N ammonia in methanol. The mixture was stirred at RT for 6h. The solvent was removed under reduce pressure. The residue was purified by silica gel flash chromatography using a linear gradient of ammonia 1 N in methanol (0→10% MeOH/NH₃) in DCM to give **20** as a white amorphous solid (32 mg; 48 µmol; 46%).
**¹H NMR (500MHz; DMSO) δ** 8.27 (s, 1H, Ha5), 8.12 (s, 1H, Ha1), 8.01 (dd, *J*=1.2, 7.8Hz, 1H, HNos), 7.95 (dd, *J*=1.2, 7.8Hz, 1H, HNos), 7.91 (ddd, *J*=1.2,7.8,15.5Hz, 1H, HNos), 7.80 (ddd, *J*=1.2, 7.8, 15.5Hz, 1H, HNos), 7.66 (brs, 1 H, HNH2c), 7.56 and 7.46 (s and d, J= 1.1 Hz, 0.82 and 0.28H, Hc3), 7.33 (brs, 2H, HNH2a), 6.36 (brs, 1H, HNH2c), 6.02 (d, *J*=2.7Hz, 1H, Ha6), 5.37 (dd, *J*=2.7, 6.4Hz, 1 H, Ha7), 4.98 and 4.93 (s and dd, *J*=9.9,13.5Hz, 2H, Hc6), 4.80 (dd, *J*=4.0, 6.3Hz, 1H, Ha8), 4.22 (d, *J*=15.9Hz, 1H, Hc1), 4.15 (d, *J*=15.9Hz, 1 H, Hc1), 3.99-3.94 (m, 1 H, Ha9), 3.32-3.29 (m, 2H, Ha11), 3.22 and 3.16 (s and s, 0.73 and 2.27H, Hc7), 1.90-1.75 (m, 1H, Ha10), 1.50 (s, 3H, HMe), 1.30 (s, 3H, HMe).
**¹³C NMR (125MHz; DMSO) δ** 164.8 (Cc5), 156.6 (Ca2), 155.6 (Cc4), 153.4 (Ca1), 149.2 (Ca4), 147.9 (CNos), 146.0 and 143.0 (Cc3), 140.3 (Ca5), 135.4 (CNos), 133.0 (CNos), 131.26 (CNos), 130.1 (CNos), 125.0 (CNos), 119.6 (Ca3), 114.2 (Cipr), 100.1 (Cc2), 88.7 (Ca6), 83.6 (Ca8), 83.5 (Ca7), 83.3 (Ca9), 78.9 and 78.6 (Cc6), 56.3 and 56.2 (Cc7), 46.3 (Cc1), 45.1 (Ca11), 32.9 (Ca10), 27.5 (CiPr), 25.7 (CiPr)
**HRMS-ESI(m/z)** calculated for C₂₃H₃₃N₁₀O₉S [M+H]⁺ : 673.2147 ; Found: 673.2102.

### 4.3. 1-(methoxymethyl)-5-((2',3'-O-isopropylidene-5'-deoxyadenosin-5'-yl) aminomethyl)cytosine (21)

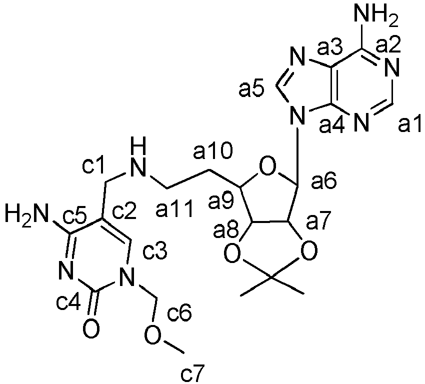

To a solution of **21** (30 mg; 45 µmol) in DMF (2 mL) was added K₂CO₃ (37 mg; 270 µmol) and thiophenol (25 µL; 250 µmol) was added and the mixture was stirred at RT overnight. The mixture was filtrated and the solvent was removed. The residue was purified by reversed phase HPLC using a linear acetonitrile gradient (0→60% CH₃CN) to obtain **22** as colourless foam (17 mg; 35 µmol; 77%).
**¹H NMR (500MHz; DMSO) δ** 8.32 (s, 1H, Ha5), 8.17 (s, 1H, Ha1), 7.47 (s, 1H, Hc3), 7.33 (brs, 2H, HNH2a), 7.13 (brs, 1 H, HNH), 6.09 (d, *J*=2.7Hz, 1 H, Ha6), 5.37 (dd, *J*=2.7, 6.4Hz, 1 H, Ha7), 4.97 (s, 2H, Hc6), 4.89(dd, *J*=3.8, 6.4Hz, 1 H, Ha8), 4.18-4.15 (m, 1 H, Ha9), 3.34 (s, 2H, Hc1), 3. 21 (s 3H, Hc7), 2.48-2.47 (m, 2H, Ha11), 1.80-1.71 (m, 1 H, Ha10), 1.53 (s, 3H, HiPr), 1.33 (s, 3H, HiPr).
**¹³C NMR (125MHz; DMSO) δ** 166.2 (Cc5), 156.6 (Ca2), 156.1 (Cc4), 153.2 (Ca1), 149.4 (Ca4), 143.4 (Cc3), 140.4 (Ca5), 119.5 (Ca3), 114.0 (Cipr), 104.1 (Cc2), 88.8 (Ca6), 84.5 (Ca8), 84.1 (Ca7), 83.5 (Ca9), 78.8 (Cc6), 56.2 (Cc7), 47.2 (Cc1), 45.1 (Ca11), 33.5 (Ca10), 27.5 (CiPr), 25.7 (CiPr)
**HRMS-ESI(m/z)** calculated for C₂₁H₃₀N₉O₅ [M+H]⁺ : 488.2364 ; Found: 488.2362.

### 4.4. 1-(methoxymethyl)-5-(N-(5'-deoxyadenosin-5'-yl)aminomethyl)cytosine (C)

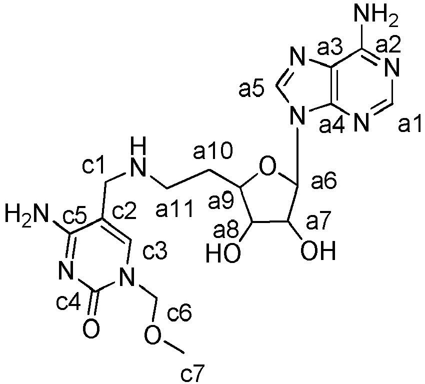

Water (50 µL) was added to a solution of **21** (15 mg; 31 µmol) in TFA (0.5 mL). The reaction mixture was stirred at RT for 1 h. The solvent was removed and the residue was co-evaporated three times with 1 N ammonia in methanol under reduce pressure to obtain **C** as a colourless foam powder (9.2 mg; 19 µmol; 89%).
**¹H NMR (500MHz; DMSO) δ** 8.57 (brs, 2H, HNH2), 8.40 (s, 1 H, Ha5), 8.24 (s, 1 H, Ha1), 7.98 (s, 1 H, Hc3), 7.72 (brs, 1 H, HNH), 5.90 (d, *J*=4.23Hz, 1 H, Ha6), 5.06 (s, 2H, Hc6), 4.62 (t, *J*=4.7Hz, 1 H, Ha7), 4.20 (t, *J*=5.4Hz, 1 H, Ha8), 3.98-3.91 (m, 2H, Ha9 and Hc1), 3.29 (s 3H, Hc7), 3. 09-3.01 (m, 2H, Ha11), 2.08-1.97 (m, 1H, Ha10).
**¹³C NMR (125MHz; DMSO) δ** 162.6 (Cc5), 155.4 (Ca2), 151.7 (Ca1), 150.6 (Cc3), 149.4 (Ca4), 140.4 (Ca5), 119.6 (Ca3), 97.3 (Cc2), 88.8 (Ca6), 81.0 (Ca9), 79.3 (Cc6), 73.6 (Ca7), 73.5 (Ca8), 56.9 (Cc7), 44.2 (Ca11), 43.2 (Cc1), 29.9 (Ca10).
**HRMS-ESI(m/z)** calculated for C₁₈H₂₀N₉O₅ [M+H]⁺ : 488.2051 ; Found: 488.2052.

### 4.5. tert-butyl 2-((Boc)amino)-4-(((2',3'-O-isopropylidene-N⁴-benzoyl-5'-deoxyadénosiny-5'-yl) methylamino)butanoate (22)

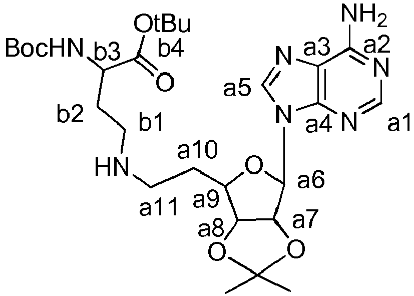

To a solution of **18** (110mg, 217µmol) in anhydrous DMF (1mL) was added K₂CO₃ (90mg, 653µmol) and (S)-2-tert-butoxycarbonylamino-4-bromotyric acid tert-butyl ester (110mg, 326µmol). After stirring at 65°C for 6h, thiophenol (50 µL; 500 µmol) was added and the mixture was stirred at RT overnight. The reaction mixture was diluted with ethyl acetate (20mL) and washed 3 times with 1 M NaHCO₃ (20mL) and brine then dried over Na₂SO₄. The solvent was removed by vacuum and the residue was purified by silica gel chromatography using a linear gradient of methanol (0→10% MeOH) in DCM to afford **22** as a white amorphous solid (66 mg; 115 µmol; 53%).
**¹H NMR (500MHz; DMSO) δ** 8.32 (s, 1H, Ha5), 8.17 (s, 1H, Ha1), 7.32 (brs, 2H, HNH2), 7.32 (bd, *J*=7.6Hz, 2H, HNH), 6.09 (d, *J*=2.7Hz, 1 H, Ha6), 5.47 (dd, *J*=2.7, 6.4Hz 1 H, Ha7), 4.88 (dd, *J*=3.7, 6.4Hz, 1 H, Ha8), 4.19-4.15 (m, 1 H, Ha9), 3.90-3.84 (m, 1 H, Hb3), 2.49-2.42 (m, 4H, Hb1 and Ha11), 1.77-1.57 (m, 4H, Ha10 and Hb2), 1.58 (m, 3H, HiPr), 1.40-1.34 (m, 18H, Ht-Bu and HBoc), 1.32 (m, 3H, HiPr).
**¹³C NMR (125MHz; CCl₃) δ** 172.4 (Cb4), 156.7 (Ca2), 155.9 (CBoc), 153.2 (Ca1), 149.4 (Ca4), 140.3 (Ca5), 119.5 (Ca3), 113.9 (CiPr), 88.7 (Ca6), 84.3 (Ca9), 84.1 (Ca7), 83.5 (Ca8), 80.5 (CtBu), 78.4 (CBoc), 53.2 (Cb3), 46.2 (Ca11), 45.8 (Cb1), 33.8 (Ca10), 31.2 (Cb2), 28.6 (CBoc), 28.1 (CtBu), 27.5 (CiPr), 25.7 (CiPr).
**HRMS-ESI(m/z)** calculated for C₂₇H₄₄N₇O₇ [M+H] : 578.3297; Found: 578.3301.

### 4.6. tert-butyl 2-((Boc)amino)-4-(((1-(methoxymethyl)-cytosin-5-yl)methyl)(5'-(2',3'-isopropylidene-5'-deoxy-adenosin-5'-yl)methyl)amino)butanoate (23)

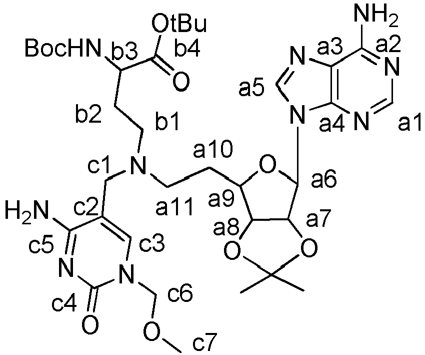

Freshly prepared **5** (125 mg; 0.35 mmol) was added to a solution of **22** (60 mg; 104 µmol) in dry acetonitrile (3 mL) and DIPEA (100 µL). After 4h at RT, the solvent was removed and the residue was diluted with ethyl acetate. The organic phase was washed with 1M NaHCO₃ and brine, dried on magnesium sulphate and the solvent was removed by vacuum. The residue was dissolved in 7N ammonia in methanol and stirred for 3h at RT. Then the solvent was removed and the residue was purified by silica gel flash chromatography using a linear gradient of ammonia 1 N in methanol (0→10% MeOH/NH₃) in DCM then by reversed phase HPLC using a linear acetonitrile gradient with 0.2% of TEA (0→80% CH₃CN) to afford **23** as a white powder (34 mg; 46 µmol; 44%).
**¹H NMR (500MHz; DMSO) δ** 8.30 (s, 1 H, Ha5), 8.16 (s, 1 H, Ha1), 7.52 (s, 1 H, Hc3), 7.32 (brs, 3H, HNH), 7.32 (bd, *J*=7.6Hz, 1H, HNH), 6.86-6.77 (m, 1H, HNH), 6.08 (d, *J*=2.6Hz, 1 H, Ha6), 5.46 (dd, *J*=2.6, 6.3Hz 1 H, Ha7), 4.97 (s, 2H, Hc6), 4.84 (dd, *J*=3.7, 6.3Hz, 1 H, Ha8), 4.11-4.01 (m, 1 H, Ha9), 3.83-3.75 (m, 1 H, Hb3), 3.27-3.14 (m, 4H, Hc7 and Hc1), 2.48-2.32 (m, 4H, Hb1 and Ha11), 1.87-1.58 (m, 4H, Ha10 and Hb2), 1.52 (m, 3H, HiPr), 1.37-1.32 (m, 18H, Ht-Bu and HBoc), 1.31 (m, 3H, HiPr).
**¹³C NMR (125MHz; CCl₃) δ** 172.1 (Cb4), 166.1 (Cc5), 156.6 (Ca2), 156.0 (CBoc), 155.9 (Cc4), 153.2 (Ca1), 149.3 (Ca4), 144.3 'Cc3), 140.4 (Ca5), 119.6 (Ca3), 114.9 (CiPr), 102.7 (Cc2), 88.8 (Ca6), 84.5 (Ca9), 84.0 (Ca7), 83.5 (Ca8), 80.7 (CtBu), 78.8 (Cc6), 78.5 (CBoc), 56.2 (Cc7), 53.0 (Cb3), 52.9 (Cc1), 49.5 (Ca11), 48.9 (Cb1), 29.8 (Cb2), 28.6 (CBoc), 28.0 (CtBu), 27.9 (Ca10), 27.5 (CiPr), 25.7 (CiPr).
**HRMS-ESI(m/z)** calculated for C₃₄H₅₃N₁₀O₉ [M+H] : 745.3991; Found: 745.3984.

### 4.7. 2-amino-4-(((1-(methoxymethyl)-cytosin-5-yl)methyl)(5'-(2',3'-isopropylidene-5'-deoxy-adenosin-5'-yl)methyl)amino)butanoic acid (D)

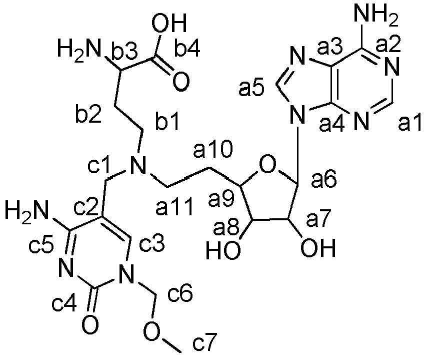

Water (50 µL) was added to a solution of **23** (30 mg; 40 µmol) in TFA (0.5 mL). The reaction mixture was stirred at RT for 1 h. The solvent was removed and the residue was co-evaporated three times with 1 N ammonia in methanol under reduce pressure to obtain **D** as a colourless foam powder (19 mg; 35 µmol; 87%).
**¹H NMR (500MHz; DMSO) δ** 9.05 (brs, 1 H, HNH), 8.42 (s, 1 H, Ha5), 8.28 (s, 1H, Ha1), 8.02 (s, 1H, Hc3), 7.98 (brs, 2H, HNH), 8.89 (d, *J*=5.3Hz, 1H, Ha6), 5.06 (s, 2H, Hc6), 4.67 (t, *J*=5.3Hz 1H, Ha7), 4.10 (t, *J*=4.8Hz, 1H, Ha8), 398-3.88 (m, 2H, Ha9 and Hb3), 3.83-3.65 (m, 2H, Hc1), 3.28 (m, 4H, Hc7), 2.98-2.78 (m, 4H, Hb1 and Ha11), 2.11-1.89 (m, 4H, Ha10 and Hb2),.
**¹³C NMR (125MHz; CCl₃) δ** 171.1 (Cb4), 161.5 (Cc5), 154.7 (Ca2), 150.8 (Ca1), 149.4 (Ca4), 141.3 (Ca5), 119.6 (Ca3), 88.8 (Ca6), 82.4 (Ca9), 79.3 (Cc6), 73.8 (Ca8), 73.4 (Ca7), 56.9 (Cc7), 50.7 (Cb3), 52.9 (Cc1), 49.5 (Ca11), 48.9 (Cb1), 29.8 (Cb2), 25.6 (Ca10),
**HRMS-ESI(m/z)** calculated for C₂₂H₃₃N₁₀O₇ [M+H] : 546.2528; Found: 546.2531.

### 5. Compounds E and F

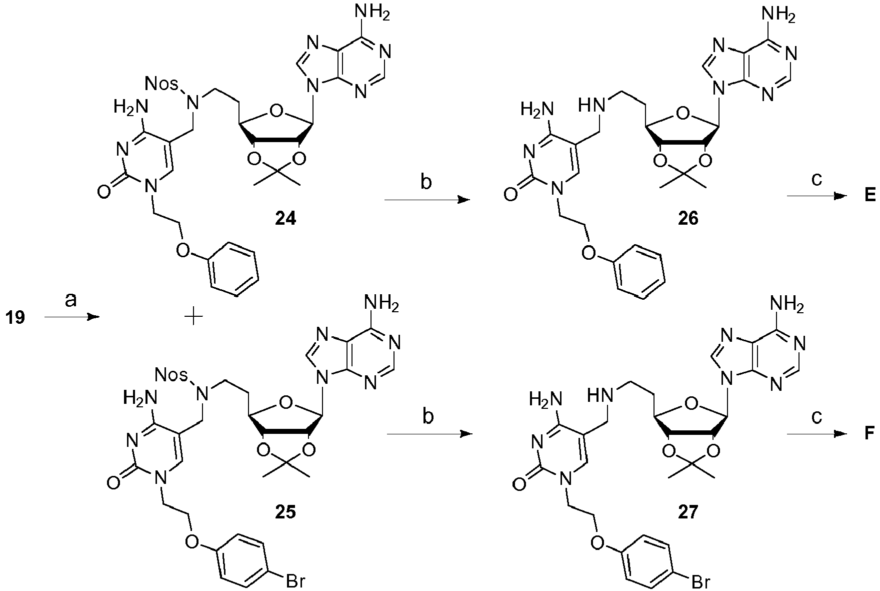

***Scheme 5.*** Synthesis of compounds **E** and **F.** a) *i)* mixture of **8** and **9,** DMF, K₂CO₃, RT, 1h, 32% and 22%. *ii*) NH₃/MeOH. b) PhSH, K₂CO₃, DMF, RT, 18h, **75** and **74** %. c) TFA, water, RT, 1 h, 75%.

Analogs **E** and **F** were obtained starting from the key synthon **19** that was produced by nosylation of 5'-aminomethyl-5'-deoxy-2',3'-*O-*isopropylideneadenosine **18** previously described in Lerner *et al.* (2003). *N-*alkylation of adenosine derivative **19** by the mixture of **8** and by-product **9** followed by treatment with thiophenol allowed the separation of compounds **24** and **25,** which gave analogs **E** and **F,** respectively, after deprotection in acidic conditions.

### 5.1. 2',3'-O-isopropylidene-5'-deoxy-5'-(N-((1-(2-phenoxyethyl)-cytosin-5-yl)methyl)-2-nitrophenylsulfonamido)methyl)adenosine (24) and

### 5.2. 2',3'-O-isopropylidene-5'-deoxy-5'-(N-((1-(2-(4-bromophenoxy)ethyl)-cytosin-5-yl)methyl)-2-nitrophenylsulfonamido)methyl)adenosine (25)

To a solution of derivative **20** (50 mg; 99 µmol) in dry DMF (2 mL) was added K₂CO₃ (41 mg; 306 µmol) and freshly prepared mixture of **8** and **9** (277 µmol). The mixture was stirred for 6h to 8h at RT (the reaction was monitored by TLC) then diluted with ethyl acetate and washed with water. The organic phase was washed with brine and dried with magnesium sulphate. Solvent was removed and the residue was dissolved in 7N ammonia in methanol. The mixture was stirred at RT for 6h. The solvent was removed under reduce pressure. The residue was purified by silica gel flash chromatography using a linear gradient of ammonia 1N in methanol (0→10% MeOH/NH₃) in DCM then by reversed phase HPLC using a linear acetonitrile gradient with 0.2% of TEA (0→80% CH₃CN) to afford **24** (24 mg; 32 µmol; 32%) and its bromide derivative **25** (18 mg; 22 µmol; 22%) as pale yellow powders. 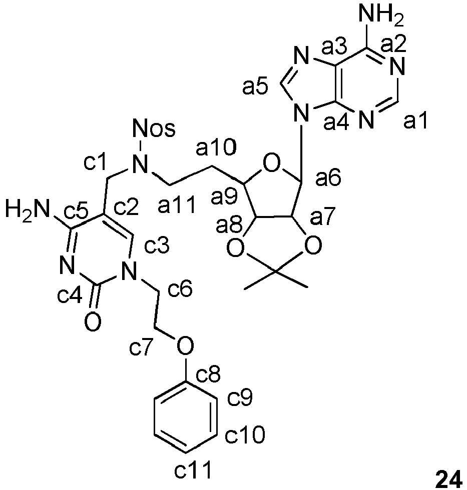 **¹H NMR (500MHz; DMSO) δ** 8.27 (s, 1H, Ha5), 8.12 (s, 1H, Ha1), 8.00 (dd, *J*=1.1, 8.1 Hz, 1 H, HNos), 7.94 (dd, *J*=1.1, 8.1 Hz, 1 H, HNos), 7.90 (ddd, *J*=1.2, 7.8, 15.5Hz, 1H, HNos), 7.76 (ddd, *J*=1.2, 7.8, 15.5Hz, 1H, HNos), 7.58 (s 1H, Hc3), 7.34 (brs, 2H, HNH), 7.27-7.22 (m, 2H, Hc10), 6.94-6.87 (m, 2H, Hc9 and Hc11), 6.01 (d, *J*=2.7Hz, 1 H, Ha6), 5.35 (dd, *J*=2.7, 6.4Hz, 1 H, Ha7), 4.77 (dd, *J*=3.9, 6.3Hz, 1 H, Ha8), 4.24-4.10 (m, 4H, Hc1 and Hc7), 3.98-3.91 (m, 3H, Ha9 and Hc6), 3.32-3.29 (m, 2H, Ha11), 1.90-1.76 (m, 2H, Ha10), 1.49 (s, 3H, HMe), 1.28 (s, 3H, HMe).
**¹³C NMR (125MHz; DMSO) δ** 164.7 (Cc5), 158.5 (Cc8), 156.6 (Ca2), 155.6 (Cc4), 153.2 (Ca1), 149.2 (Ca4), 147.9 (CNos), 147.7 (Cc3), 140.3 (Ca5), 135.3 (CNos), 132.9 (CNos), 131.4 (CNos), 130.1 (CNos),129.9 (Cc10), 124.9 (CNos), 121.1 (Cc11), 119.6 (Ca3), 114.8 (Cc9), 114.1 (Cipr), 99.0 (Cc2), 88.7 (Ca6), 83.6 (Ca7), 83.5 (Ca8), 83.3 (Ca9), 65.6 (Cc7), 48.9 (Cc6), 46.1 (Cc1), 45.0 (Ca11), 32.6 (Ca10), 27.5 (CiPr), 25.7 (CiPr)
**HRMS-ESI(m/z)** calculated for C₃₃H₃₇N₁₀O₉S [M+H]⁺ : 749.2460 ; Found: 749.2462. 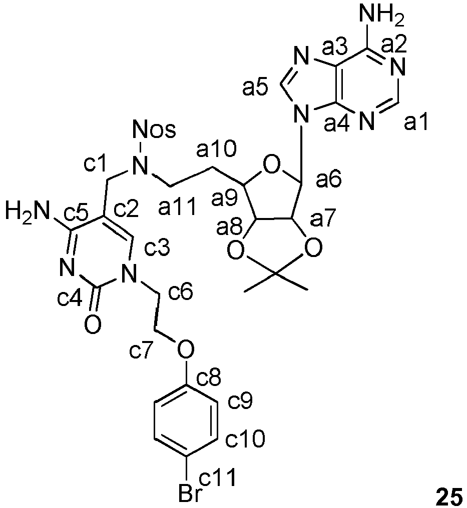 **¹H NMR (500MHz; DMSO) δ** 8.27 (s, 1H, Ha5), 8.11 (s, 1H, Ha1), 8.00 (dd, *J*=1.2, 8.0Hz, 1 H, HNos), 7.94 (dd, *J*=1.2, 8.0Hz, 1 H, HNos), 7.90 (ddd, *J*=1.2, 7.7, 15.5Hz, 1H, HNos), 7.78 (ddd, *J*=1.2, 7.8, 15.5Hz, 1H, HNos), 7.56 (s 1H, Hc3), 7.40 (d, *J*=8.9Hz, 2H, Hc10), 7.34 (brs, 2H, HNH), 6.88 (d, *J*=8.9Hz, 2H, Hc9), 6.01 (d, *J*=2.6Hz, 1H, Ha6), 5.35 (dd, *J*=2.6, 6.5Hz, 1H, Ha7), 4.76 (dd, *J*=4.0, 6.5Hz, 1H, Ha8), 4.23-4.09 (m, 4H, Hc1 and Hc7), 3.978-3.92 (m, 3H, Ha9 and Hc6), 3.32-3.29 (m, 2H, Ha11), 1.90-1.77 (m, 2H, Ha10), 1.49 (s, 3H, HMe), 1.28 (s, 3H, HMe).
**¹³C NMR (125MHz; DMSO) δ** 164.7 (Cc5), 157.8 (Cc8), 156.6 (Ca2), 155.6 (Cc4), 153.2 (Ca1), 149.2 (Ca4), 147.9 (CNos), 147.6 (Cc3), 140.3 (Ca5), 135.3 (CNos), 133.0 (CNos), 132.5 (Cc10),131.4 (CNos), 130.1 (CNos), 124.9 (CNos), 119.6 (Ca3), 117.1 (Cc9), 114.1 (Cipr), 112.7 (Cc11), 99.1 (Cc2), 88.7 (Ca6), 83.6 (Ca7), 83.5 (Ca8), 83.3 (Ca9), 66.0 (Cc7), 48.7 (Cc6), 46.1 (Cc1), 45.1 (Ca11), 32.7 (Ca10), 27.5 (CiPr), 25.7 (CiPr)
**HRMS-ESI(m/z)** calculated for C₃₃H₃₆N₁₀O₉SBr [M+H]⁺ : 827.1565 ; Found: 827.1571.

### 5.3. 2',3'-O-isopropylidene-5'-deoxy-5'-(N-((1-(2-(4-phenoxy)ethyl)-cytosin-5-yl)methyl)-aminomethyl)adenosine (26) and

### 5.4. 2',3'-O-isopropylidene-5'-deoxy-5'-(N-((1-(2-(4-bromophenoxy)ethyl)-cytosin-5-yl)methyl)-aminomethyl)adenosine (27)

To a solution of **24** (22 mg; 29.4 µmol) or **25** (15 mg; 18.2 µmol) and K₂CO₃ (3 eq.) in acetonitrile (0.5mL) was added thiophenol (50 µL; 500 µmol) and the mixture was stirred at RT overnight. The reaction mixture was diluted with ethyl acetate (20 mL) and washed 3 times with 1M NaHCO₃ (20mL) and brine then dried over Na₂SO₄. The solvent was removed by vacuum and the residue was purified by silica gel chromatography using a linear gradient of methanol (0→10% MeOH) in DCM. **26** (12 mg; 22.1 µmol; 75%) and **27** (8.5 mg; 13.2 µmol; 73%) were obtained as white powders. 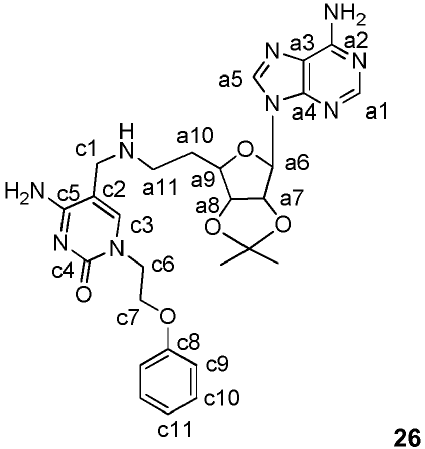 **¹H NMR (500MHz; DMSO) δ** 8.33 (s, 1 H, Ha5), 8.18 (s, 1 H, Ha1), 7.49 (s 1 H, Hc3), 7.34 (brs, 2H, HNH), 7.28-7.23 (m, 2H, Hc10), 7.15 (brs, 1 H, HNH), 6.98 (brs, 1 H, HNH), 6.95-6.88 (m, 2H, Hc9 and Hc11), 6.01 (d, *J*=2.6Hz, 1 H, Ha6), 5.48 (dd, *J*=2.6, 6.4Hz, 1H, Ha7), 4.90 (dd, J=3.9, 6.3Hz, 1H, Ha8), 4.20-4.10 (m, 3H, H9 and Hc7), 3.98 (d, *J*=5.5Hz, 1 H, Hc6), 3.31-3.28 (m, 3H, Hc1), 2.49-2.45 (m, 2H, Ha11), 1.81-1.70 (m, 2H, Ha10), 1.53 (s, 3H, HMe), 1.32 (s, 3H, HMe).
**¹³C NMR (125MHz; DMSO) δ** 166.0 (Cc5), 158.5 (Cc8), 156.6 (Ca2), 156.0 (Cc4), 153.2 (Ca1), 149.4 (Ca4), 145.1 (Cc3), 140.4 (Ca5), 129.9 (Cc10), 121.2 (Cc11), 119.5 (Ca3), 114.8 (Cc9), 113.9 (Cipr), 103.1 (Cc2), 88.8 (Ca6), 84.5 (Ca9), 84.1 (Ca8), 83.5 (Ca9), 65.6 (Cc7), 48.6 (Cc6), 47.2 (Cc1), 44.9 (Ca11), 33.6 (Ca10), 27.5 (CiPr), 25.7 (CiPr)
**HRMS-ESI(m/z)** calculated for C₂₇H₃₄N₉O₅ [M+H]⁺ : 564.2677 ; Found: 564.2681. 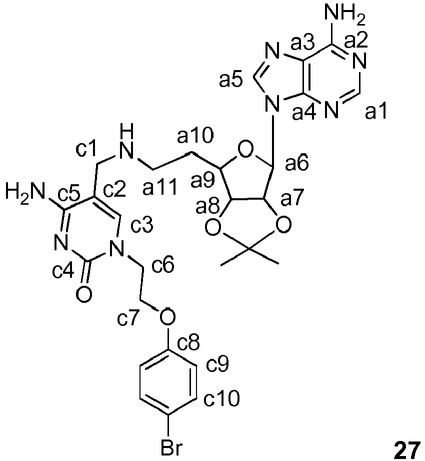 **¹H NMR (500MHz; DMSO) δ** 8.33 (s, 1 H, Ha5), 8.17 (s, 1 H, Ha1), 7.47 (s 1 H, Hc3), 7.34 (brs, 2H, HNH), 7.42 (d, *J*=9.1 Hz, 2H, Hc10), 7.34 (brs, 2H, HNH), 6.89 (d, *J*=9.1 Hz, 2H, Hc9), 6.10 (d, *J*=2.7Hz, 1 H, Ha6), 5.48 (dd, *J*=2.7, 6.4Hz, 1H, Ha7), 4.89 (dd, *J*=3.6, 6.4Hz, 1H, Ha8), 4.20-4.11 (m, 3H, Ha9 and Hc7), 3.97 (t, *J*=*5*.*1*Hz, 2H, Hc6), 3.33 (m, 2H, Hc1), 2.47 (t, *J*=6.9Hz, 2H, Ha11), 1.81-1.70 (m, 2H, Ha10), 1.53 (s, 3H, HMe), 1.32 (s, 3H, HMe).
**¹³C NMR (125MHz; DMSO) δ** 166.0 (Cc5), 157.9 (Cc8), 156.6 (Ca2), 156.0 (Cc4), 153.2 (Ca1), 149.2 (Ca4), 145.1 (Cc3), 140.3 (Ca5), 132.6 (Cc10), 119.6 (Ca3), 114.8 (Cc9), 113.9 (Cipr), 112.6 (Cc11), 103.1 (Cc2), 88.8 (Ca6), 84.5 (Ca7), 84.0 (Ca8), 83.5 (Ca9), 66.1 (Cc7), 48.4 (Cc6), 47.1 (Cc1), 45.0 (Ca11), 33.6 (Ca10), 27.5 (CiPr), 25.7 (CiPr)
**HRMS-ESI(m/z)** calculated for C₂₇H₃₃N₉O₅Br [M+H]⁺ : 642.1783 ; Found: 642.1783.

### 5.5. 5'-deoxy-5'-(N-((1-(2-(4-phenoxy)ethyl)-cytosin-5-yl)methyl)-aminomethyl) adenosine (E) and

### 5.6. 5'-deoxy-5'-(N-((1-(2-(4-bromophenoxy)ethyl)-cytosin-5-yl)methyl)-aminomethyl) adenosine (F)

Water (50 µL) was added to a solution of **26** (12 mg; 22.1 µmol) or **27** (8.5 mg; 13.2 µmol) in TFA (0.5 mL). The reaction mixtures were stirred at RT for 1 h. The solvent was removed and the residues were co-evaporated three times with 1 N ammonia in methanol under reduce pressure. The residues were purified by reversed phase HPLC using a linear acetonitrile gradient with 0.2% of TEA (0→80% CH₃CN) to afford **E** (9.1 mg; 22.1 µmol; 75%) and **F** (6.3 mg; 22.1 µmol; 75%) respectively as colourless foam powders. 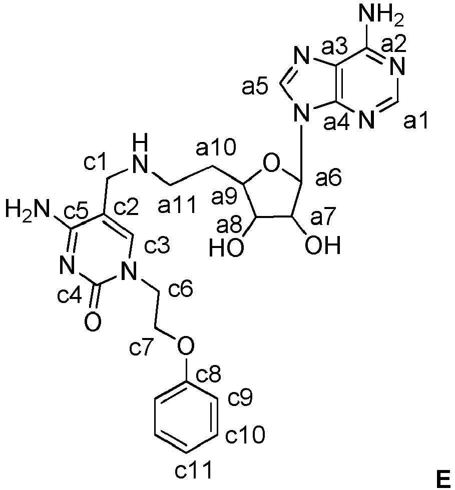 **¹H NMR (500MHz; DMSO) δ** 8.31 (s, 1 H, Ha5), 8.16 (s, 1 H, Ha1), 7.51 (s 1 H, Hc3), 7.32-7.22 (m, 3H, HNH and Hc10), 7.15 (brs, 1H, HNH), 7.08 (brs, 1H, HNH), 6.95-6.87 (m, 2H, Hc9 and Hc11), 5.85 (d, *J*=4.8Hz, 1 H, Ha6), 5.46 (d, *J*=5.6Hz, 1 H, HNH), 5.26 (brs, 1 H, HNH), 4.66 (q, *J*=5.1 Hz, 1 H, Ha7), 4.14 (t, *J*=5.4Hz, 2H, Hc7), 4.08 (t, *J*=5.1 Hz, 1 H, Ha8), 3.99 (t, *J*=5.3Hz, 2H, Hc6), 3.93 (q, *J*=5.4Hz, 1 H, Ha9), 3.43-3.36 (m, 3H, Hc1), 2.61-2.51 (m, 2H, Ha11), 1.83 (q, *J*=6.6Hz, 2H, Ha10),
**¹³C NMR (125MHz; DMSO) δ** 166.0 (Cc5), 158.5 (Cc8), 156.5 (Ca2), 156.0 (Cc4), 153.1 (Ca1), 149.8 (Ca4), 145.2 (Cc3), 140.3 (Ca5), 130.0 (Cc10), 121.2 (Cc11), 119.6 (Ca3), 114.9 (Cc9), 103.1 (Cc2), 88.1 (Ca6), 82.6 (Ca9), 73.9 (Ca8), 73.5 (Ca9), 65.6 (Cc7), 48.6 (Cc6), 47.2 (Cc1), 45.4 (Ca11), 33.8 (Ca10),
**HRMS-ESI(m/z)** calculated for C₂₄H₃₀N₉O₅ [M+H]⁺ : 524.2364 ; Found: 524.2342. 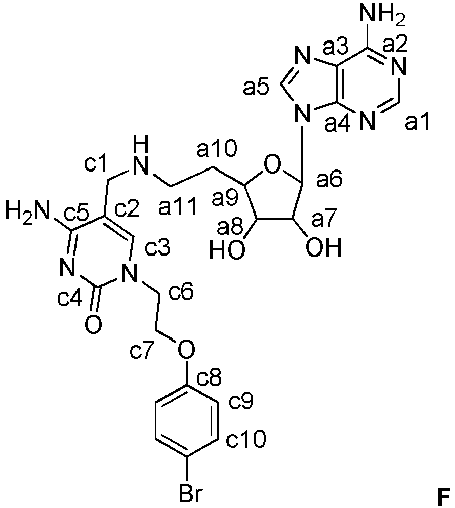 **¹H NMR (500MHz; DMSO) δ** 8.29 (brd, *J*=*16*.*1*Hz, 1H, Ha5), 8.15 (brs, 1H, Ha1), 7.49 (s 1H, Hc3), 7.45-7.39 (m, 2H, Hc10), 7.28 (brs, 1H, HNH), 7.23 (brs, 1H, HNH), 7.15 (brs, 1H, HNH), 7.08 (brs, 1H, HNH), 6.92-6.86 (d, J=9.1 Hz, 2H, Hc9), 5.89-5.76 (m, 1 H, Ha6), 5.49-42 (m, 1 H, Ha7), 4.70-4.57 (m 1 H, Ha8), 4.20-4.06 (m, 3H, Ha9 and Hc7), 4.01-3.87 (m, 3H, Hc6 and Hc1), 2.45 (q, *J*=*7.1*Hz, 2H, Ha11), 1.87-1.70 (m, 2H, Ha10).
**¹³C NMR (125MHz; DMSO) δ** 166.0 (Cc5), 157.8 (Cc8), 156.5 (Ca2), 156.0 (Cc4), 153.1 (Ca1), 149.8 (Ca4), 145.1 (Cc3), 140.3 (Ca5), 132.6 (Cc10), 119.6 (Ca3), 117.2 (Cc9), 112.6 (Cc11), 103.1 (Cc2), 88.1 (Ca6), 82.6 (Ca9), 73.9 (Ca7), 73.5 (Ca8), 66.0 (Cc7), 48.4 (Cc6), 47.1 (Cc1), 45.4 (Ca11), 33.7 (Ca10).
**HRMS-ESI(m/z)** calculated for C₂₄H₂₉N₉O₅Br [M+H]⁺ : 602.1470 ; Found: 602.1462.

### II. Biological activity

The inhibitory effect of compounds on the methyltransferase activity of human PRMT was evaluated via the measurement of its capacity to transfer methyl groups in a radioactivity assay.

### METHODS:

### - PRMT1 inhibition assay

The test compound, reference compound, or water (control) were incubated for 20 min at 22°C with 20 ng human PRMT1 enzyme, 700 nM [³H]SAM and 50 nM Histone H4 full length in a buffer containing 45 mM Tris-HCl (pH 8), 4.5 mM MgCl₂, 45 mM NaCl and 3.6 mM DTT.

For control basal measurements, the enzyme is omitted from the reaction mixture. Following incubation, the reaction is stopped by adding 33 mM citric acid and the samples are filtered rapidly under vacuum through glass fiber filters (GF/B, Packard) presoaked with 33 mM citric acid and rinsed several times with ice-cold 33 mM citric acid using a 96-sample cell harvester (Unifilter, Packard). The filters are dried then counted for radioactivity in a scintillation counter (Topcount, Packard) using a scintillation cocktail (Microscint 0, Packard).

The results are expressed as a percent inhibition of the control enzyme activity. The standard reference compound is SAH, which is tested in each experiment at several concentrations to obtain a competition curve from which its IC50 is calculated (Cheng et al., 2004, J. Biol. Chem. 279:23892).

### -PRMT4 inhibition assay

The test compound, reference compound, or water (control) were incubated for 1 h at 22°C with 2.5 ng human PRMT4 enzyme, 60 nM [³H]SAM and 25 nM Histone H3 full length in a buffer containing 45 mM Tris-HCl (pH 8), 45 mM NaCl, 4.5 mM MgCl₂ and 3.6 mM DTT.

For control basal measurements, the enzyme is omitted from the reaction mixture. Following incubation, the reaction is stopped by adding 33 mM citric acid and the samples are filtered rapidly under vacuum through glass fiber filters (GF/B, Packard) presoaked with 33 mM citric acid and rinsed several times with ice-cold 33 mM citric acid using a 96-sample cell harvester (Unifilter, Packard). The filters are dried then counted for radioactivity in a scintillation counter (Topcount, Packard) using a scintillation cocktail (Microscint 0, Packard).

The results are expressed as a percent inhibition of the control enzyme activity. The standard reference compound is SAH, which is tested in each experiment at several concentrations to obtain a competition curve from which its IC50 is calculated (Selvi et al., 2010, J. Biol. Chem., 285: 7143).

### - PRMT6 inhibition assay

The test compound, reference compound, or water (control) were incubated for 120 min at 22°C with 10 ng human PRMT6 enzyme, 120 nM [³H]SAM and 25 nM Histone H3 full length in a buffer containing 45 mM Tris-HCl (pH 8), 45 mM NaCl, 4.5 mM MgCl₂ and 3.6 mM DTT.

For control basal measurements, the enzyme is omitted from the reaction mixture. Following incubation, the reaction is stopped by adding 33 mM citric acid and the samples are filtered rapidly under vacuum through glass fiber filters (GF/B, Packard) presoaked with 33 mM citric acid and rinsed several times with ice-cold 33 mM citric acid using a 96-sample cell harvester (Unifilter, Packard). The filters are dried then counted for radioactivity in a scintillation counter (Topcount, Packard) using a scintillation cocktail (Microscint 0, Packard).

The results are expressed as a percent inhibition of the control enzyme activity. The standard reference compound is SAH, which is tested in each experiment at several concentrations to obtain a competition curve from which its IC50 is calculated. (lberg et al., 2007, J. Biol. Chem., 283: 3006).

### -PRMT7 inhibition assay

The test compound, reference compound, or water (control) were incubated for 90 min at 22°C with 12.5 ng human PRMT7 enzyme, 250 nM [³H]SAM and 4 nM Histone H2B (21-41) biotin labelled in a buffer containing 45 mM Tris-HCl (pH 8.7), 0.0045% Tween20, 0.009% HSA and 0.9 mM DTT.

For control basal measurements, the enzyme is omitted from the reaction mixture. Following incubation, the reaction is stopped by adding 33 mM citric acid and the samples are filtered rapidly under vacuum through glass fiber filters (GF/B, Packard) presoaked with 33 mM citric acid and rinsed several times with ice-cold 33 mM citric acid using a 96-sample cell harvester (Unifilter, Packard). The filters are dried then counted for radioactivity in a scintillation counter (Topcount, Packard) using a scintillation cocktail (Microscint 0, Packard).

The results are expressed as a percent inhibition of the control enzyme activity. The standard reference compound is SAH, which is tested in each experiment at several concentrations to obtain a competition curve from which its IC50 is calculated (Zurita-Lopez et al., 2012, J. Biol. Chem., 287(11):7859).

**RESULTS:**

| **Compound** | **PRMTs** | | | | | | | | |
|---|---|---|---|---|---|---|---|---|---|
| | **PRMT1** | | **PRMT4/CARM1** | | | **PRMT6** | | **PRMT7** | |
| | **100µM** | **10µM** | **100µM** | **10µM** | **IC₅₀ (µM)** | **100µM** | **10µM** | **100µM** | **10µM** |
| **A** | - | - | 21 | - | - | - | - | 12 | - |
| **B** | 24 | - | 59 | 21 | - | 61 | 3 | 81 | 30 |
| **C** | 13 | - | 99 | 81 | 1.9 | 20 | - | 89 | 33 |
| **D** | 42 | - | 88 | 54 | 14 | 47 | 8 | 83 | 46 |
| **E** | - | - | - | 35 | - | - | - | - | - |
| **F** | - | - | - | 43 | - | - | - | - | - |

The compounds of the invention thus present a high activity on PRMT. For compounds C and D, the IC50 were determined against PRMT4 resulting in potent microM inhibitors. Crystal structures of PRMT4 and PRMT6 in the presence of certain compounds confirmed the inhibition activity and revealed that the compounds could act as *in vitro* competitive inhibitors of the SAM cofactor of PRMTs.

## Claims

1. A compound of following formula (I): 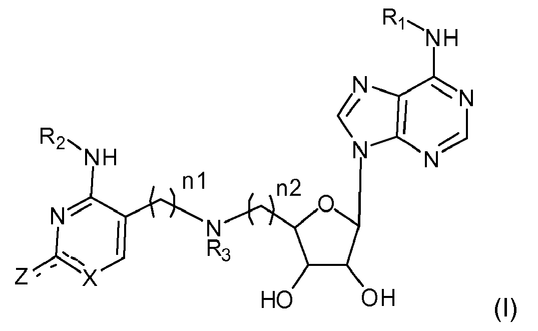 or a pharmaceutically acceptable salt and/or solvate thereof,
wherein:
- -̅-̅-̅-̅-̅-̅ represents a single bond or a double bond provided that the two adjacent -̅-̅-̅-̅-̅-̅ bonds do not represent simultaneously a double bond,
- n1 and n2 represents, independently of each other, an integer comprised between 0 and 4,
- X represents N when -̅-̅-̅-̅-̅-̅X represents a double bond -̅X or NR₄ when -̅-̅-̅-̅-̅-̅X represents a single bond X,
- Z represents O or S when -̅-̅-̅-̅-̅-̅Z represents a double bond Z or H or NR₅R₆ when -̅-̅-̅-̅-̅-̅Z represents a single bond Z,
- R₁, R₂, R₅ and R₆ represent, independently of one another, H, (C₁-C₆)alkyl, alkylaryl or arylalkyl,
- R₃ represents H, or a (C₁-C₆)alkyl optionally substituted, and
- R₄ represents H, or a (C₁-C₆ alkyl) optionally substituted with a (C₁-C₆)alkoxy, (C₁-C₆)haloalkoxy, aryloxy or haloaryloxy.

2. The compound according to claim 1, being a compound of following formula (Ia) or (Ib): 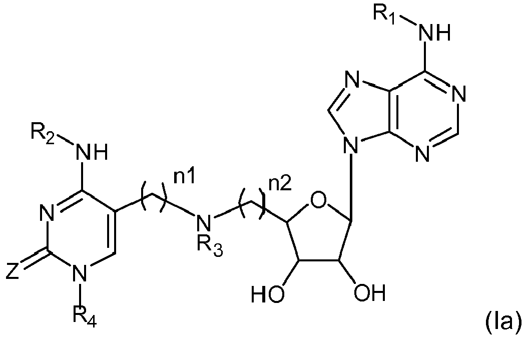 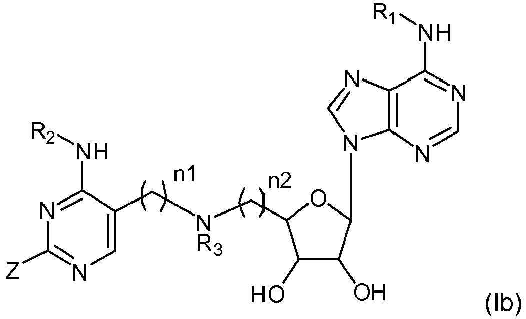 or a pharmaceutically acceptable salt and/or solvate thereof, in which n1, n2, Z, R₁, R₂, R₃ and R₄ are as defined in claim 1.

3. The compound according to claim 1, being a compound of following formula (Ic): 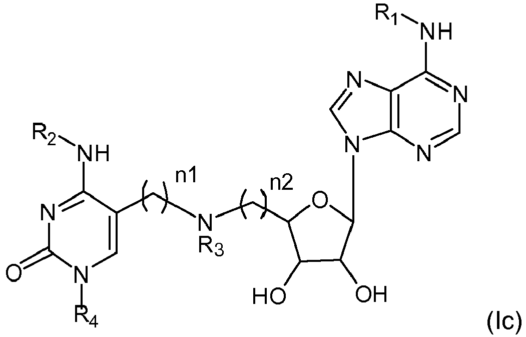 or a pharmaceutically acceptable salt and/or solvate thereof, in which n1, n2, R₁, R₂, R₃ and R₄ are as defined in claim 1.

4. The compound according to any one of claims 1 to 3, wherein the cycle 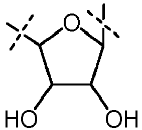 has the following stereochemistry:

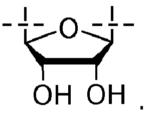

5. The compound according to any one of claims 1 to 4, wherein n1 and n2 represent, independently of one another, 1, 2, 3 or 4, notably 1 or 2.

6. The compound according to any one of claims 1 to 5, wherein R₁, R₂, R₅ and R₆ represent H.

7. The compound according to any one of claims 1 to 6, wherein R₃ represents H or a (C₁-C₆)alkyl optionally substituted with one or more substituents selected from halogen, OR₇, NR₈R₉, COR₁₀, CO₂R₁₁ and CONR₁₂R₁₃, notably selected from OR₇, NR₈R₉, COR₁₀, CO₂R₁₁ and CONR₁₂R₁₃, in particular selected from NR₈R₉ and CO₂R₁₁,
wherein R₇ to R₁₃ are, independently of one another, H or (C₁-C₆)alkyl.

8. The compound according to any one of claims 1 to 7, wherein R₄ represents H or a (C₁-C₆ alkyl) optionally substituted with a (C₁-C₆)alkoxy, aryloxy or haloaryloxy, in particular optionally substituted with a (C₁-C₆)alkoxy, aryloxy or bromoaryloxy.

9. The compound according to any one of claims 1 to 8, being selected from: 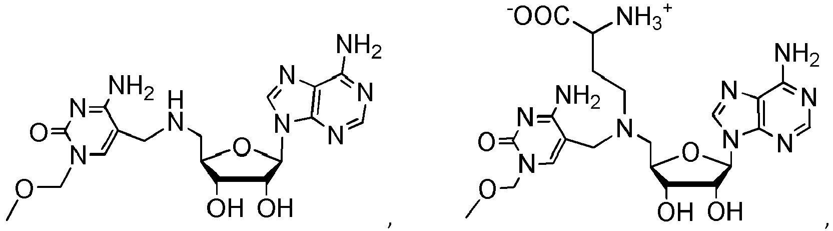 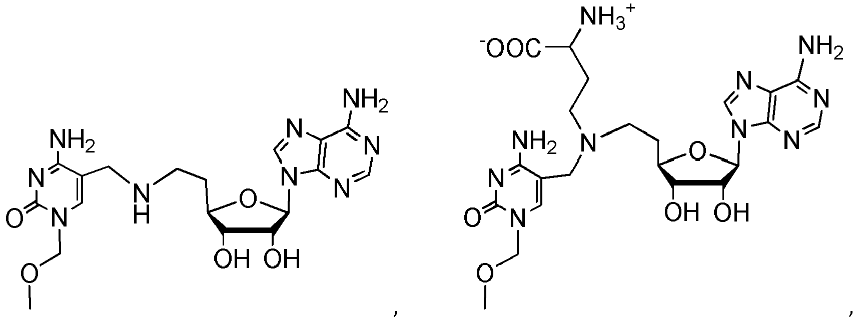 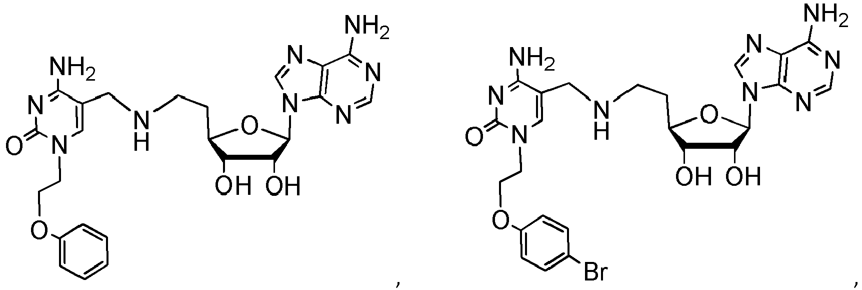 and the pharmaceutically acceptable salts and/or solvates thereof.

10. A compound according to any one of claims 1 to 9 for use as a drug.

11. A pharmaceutical composition comprising at least one compound according to any one of claims 1 to 9 and at least one pharmaceutically acceptable excipient.

12. The pharmaceutical composition according to claim 11, further comprising at least one other active ingredient, such as an anticancer agent.

13. A pharmaceutical composition comprising:
(i) at least one compound according to any one of claims 1 to 9, and
(ii) at least one other active ingredient, such as an anticancer agent, as a combination product for simultaneous, separate or sequential use.

14. A compound according to any one of claims 1 to 9 or pharmaceutical composition according to any one of claims 12 to 13 for use in the prevention and/or treatment of a PRMT-related disorder such as cancer.

15. A method for the preparation of a compound according to any one of claims 1 to 9 comprising the following steps:
(i) reacting a compound of following formula (II): 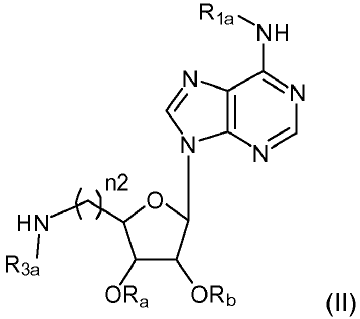 in which n2 is as defined in claim 1, R₁ₐ represents a R₁ group as defined in claim 1 optionally in a protected form, R₃ₐ represents a R₃ group as defined in claim 1 optionally in a protected form, and Rₐ and R_{b} represent, independently of one another, H or a O-protecting group,
with a compound of following formula (III): 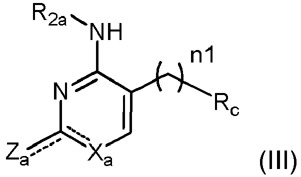 in which n1 is as defined in claim 1, R₂ₐ represents a R₂ group as defined in claim 1 optionally in a protected form, Zₐ represents a Z group as defined in claim 1 optionally in a protected form, Xₐ represents a X group as defined in claim 1 optionally in a protected form, and R_{c} represents a leaving group,
in order to obtain a compound of following formula (I-1): 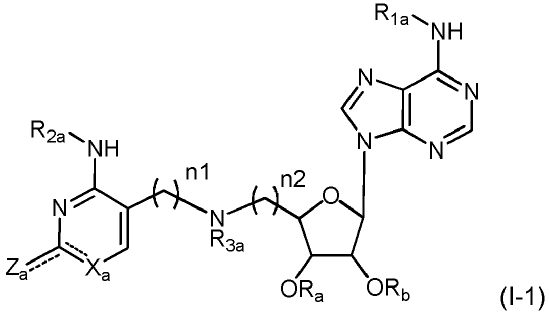 in which n1 and n2 are as defined in claim 1 and Xₐ, Zₐ, R₁ₐ, R₂ₐ, R₃ₐ, Rₐ and R_{b} are as defined above,
which is a compound of formula (I) optionally in a protected form,
(ii) when the compound of formula (I-1) is a compound of formula (I) in a protected form, deprotecting the compound of formula (I-1) in order to obtain a compound of formula (I), and
(iii) optionally salifying and/or solvating the compound of formula (I) obtained previously in order to obtain a pharmaceutically acceptable salt and/or solvate of compound of formula (I).
